# EUROPEAN PATENT APPLICATION

(11) **EP 1 481 993 A1**
(43) Date of publication of application: **01.12.2004**
(21) Application number: 03012314.5
(22) Date of filing: 28.05.2003
(51) Int. Cl.: C07K 16/28, A61K 39/395, A61P 35/00, G01N 33/574

(54) **Modulation of the poliovirus receptor function**

(71) Applicant: Xerion Pharmaceuticals AG, 81377 München (DE); Tufts University, Boston, MA 02111 (US)
(72) Inventor: Unger, Christine Margarete, 80469 München (DE); Beste, Gerald, 80687 München (DE); Zehetmeier, Carolin, 81541 München (DE); Lain, Blanca, Brighton, MA 02135 (US); Torella, Claudia, 82829 München (DE); Jay, Daniel G., Brighton, MA 02111 (US); Eustace, Brenda K., Brookline, MA 02446 (US)
(74) Representative: Fleuchaus, Andrea

(57) **Abstract**

The present invention relates to the identification, the isolation and the use of molecules interfering with the function(s) mediated by the poliovirus receptor (PVR) on cells. The molecules can be used for the treatment of cells having a metastatic potential, metastasis and cancer. Further methods are provided that are useful for identifying and isolating molecules, which have the capacity to modulate PVR mediated adhesion or invasion potential of cells.

SEQUENCE LISTING

<110> Xerion Pharmaceuticals AG
      Tufts University
<120> Modulation of the Poliovirus Receptor Function
<130> XE1EP-5744
<140>
<141>
<160> 72
<170> PatentIn Ver. 2.1

<210> 1
<211> 6
<212> PRT
<213> Artificial Sequence
<220>
<223> Description of Artificial Sequence: peptide
<400> 1
Leu Trp Leu Arg Arg Asp
  1               5

<210> 2
<211> 7
<212> PRT
<213> Artificial Sequence
<220>
<223> Description of Artificial Sequence: Peptide
<400> 2
Trp Thr Gly Asp Phe Asp Tyr
  1               5

<210> 3
<211> 268
<212> PRT
<213> Artificial Sequence
<220>
<223> Description of Artificial Sequence:  single chain antibody
<400> 3

Glu Val Gln Leu Gln Gln Ser Gly Ala Glu Leu Val Arg Pro Gly Thr
1               5                   10                  15
Ser Val Lys Leu Ser Cys Lys Ala Leu Gly Tyr Thr Phe Thr Asp His
            20                  25                  30
Glu Met His Trp Val Lys Gln Thr Pro Val His Gly Leu Glu Trp Ile
        35                  40                  45
Gly Thr Ile His Pro Gly Ser Gly Val Thr Ala Tyr Asn Gln Lys Phe
    50                  55                  60
Lys Gly Lys Ala Thr Leu Thr Ala Asp Lys Ser Ser Ser Thr Ala Tyr
65                  70                  75                  80
Met Glu Leu Ser Thr Leu Thr Ser Glu Asp Ser Ala Val Tyr Tyr Cys
                85                  90                  95
Thr Pro Leu Trp Leu Arg Arg Asp Trp Gly Gln Gly Thr Thr Leu Thr
            100                 105                 110
Val Ser Thr Gly Gly Gly Gly Ser Gly Gly Gly Gly Ser Gly Gly Gly
        115                 120                 125
Gly Ser Ala Leu Asp Ile Gln Met Thr Gln Thr Pro Lys Phe Met Ser
    130                 135                 140
Thr Ser Val Gly Asp Arg Val Ser Val Thr Cys Lys Ala Ser Gln Asn
145                 150                 155                 160
Val Ala Thr Asn Val Val Trp Phe Gln Gln Lys Ser Gly Gln Ser Pro
                165                 170                 175
Lys Ala Leu Ile Tyr Ser Ala Ser Tyr Arg Tyr Ser Gly Val Pro Asp
            180                 185                 190
Arg Phe Thr Gly Ser Gly Ser Gly Thr Asp Phe Thr Leu Thr Ile Ser
        195                 200                 205
Asn Val Gln Ser Glu Asp Leu Ala Glu Tyr Phe Cys Gln Gln Tyr Asn
    210                 215                 220
Asn Tyr Pro Leu Thr Phe Gly Ala Gly Thr Lys Leu Glu Leu Lys Ala
225                 230                 235                 240
Ala Ala Gly Ala Pro Val Pro Tyr Pro Asp Pro Leu Glu Pro Arg Gly
                245                 250                 255
Ala Ala Ser Ala Trp Ser His Pro Gln Phe Glu Lys
            260                 265

<210> 4
<211> 267
<212> PRT
<213> Artificial Sequence

<220>
<223> Description of Artificial Sequence: single chain antibody
<400> 4
Gln Leu Gln Gln Ser Gly Pro Glu Leu Val Lys Pro Gly Ala Ser Val
1               5                   10                  15
Lys Ile Ser Cys Lys Thr Ser Gly Tyr Thr Phe Thr Glu Tyr Thr Met
            20                  25                  30
His Trp Val Lys Gln Ser His Gly Lys Ser Leu Glu Trp Ile Gly Gly
        35                  40                  45
Ile His Pro Asn Asn Gly Asp Thr Ser Tyr Asn Gln Arg Phe Lys Gly
    50                  55                  60
Lys Ala Thr Leu Thr Val Asp Lys Ser Ser Ser Thr Ala Tyr Met Glu
65                  70                  75                  80
Leu Arg Ser Leu Thr Ser Glu Asp Ser Ala Val Tyr Tyr Cys Ala Arg
                85                  90                  95
Trp Thr Gly Asp Phe Asp Tyr Trp Gly Gln Gly Thr Thr Leu Thr Val
            100                 105                 110
Ser Thr Gly Gly Gly Gly Ser Gly Gly Gly Gly Ser Gly Gly Gly Gly
        115                 120                 125
Ser Ala Leu Asp Ile Val Met Thr Gln Ser Pro Lys Phe Met Ser Thr
    130                 135                 140
Ser Val Gly Asp Arg Val Ser Val Thr Cys Lys Ala Ser Gln Asn Val
145                 150                 155                 160
Gly Thr Asn Val Ala Trp Tyr Gln Gln Lys Pro Gly Gln Ser Pro Lys
                165                 170                 175
Ala Leu Ile Tyr Ser Ala Ser Tyr Arg Tyr Ser Gly Val Pro Asp Arg
            180                 185                 190
Phe Thr Gly Ser Gly Ser Gly Thr Asp Phe Thr Leu Thr Ile Ser Asn
        195                 200                 205
Val Gln Ser Glu Asp Leu Ala Glu Tyr Phe Cys Gln Gln Tyr Asn Ser
    210                 215                 220
Tyr Pro Tyr Thr Phe Gly Gly Gly Thr Lys Leu Glu Ile Lys Ala Ala
225                 230                 235                 240
Ala Gly Ala Pro Val Pro Tyr Pro Asp Pro Leu Glu Pro Arg Gly Ala
                245                 250                 255
Ala Ser Ala Trp Ser His Pro Gln Phe Glu Lys
            260                 265

<210> 5

<211> 807
<212> DNA
<213> Artificial Sequence
<220>
<223> Description of Artificial Sequence: single chain antibody
<400> 5
gaggttcagc tgcagcagtc tggggctgag ctggtgaggc ctgggacttc agtgaagctg 60
tcctgcaagg ctttgggcta cacatttact gaccatgaaa tgcactgggt gaaacagaca 120
cctgtgcatg gcctggaatg gattggaact attcatccag gaagtggtgt tactgcctac 180
aatcagaagt tcaagggcaa ggccacactg actgcagaca aatcctccag cacagcctac 240
atggagctca gcaccctgac atctgaagac tctgctgtct attactgtac accactttgg 300
ttacgacggg actggggcca aggcaccact ctcacagtgt cgacaggtgg aggcggttca 360
ggcggaggtg gctctggcgg tggcggaagt gcactcgata tccagatgac acagactcca 420
aaattcatgt ccacatcagt aggagacagg gtcagcgtca cctgcaaggc cagtcagaat 480
gtggctacta atgtagtctg gtttcaacag aaatcagggc aatctcctaa agcactgatt 540
tactcggcat cctaccggta cagtggagtc cctgatcgct tcacaggcag tggatctggg 600
acagatttca ctctcaccat cagcaatgtg cagtctgaag acttggcaga gtatttctgt 660
cagcaatata acaactatcc tctcacgttc ggtgctggga ccaagctgga gctgaaagcg 720
gccgcaggtg cgccggtgcc gtatccagat ccgctggaac cgcgtggggc cgcaagcgct 780
tggagccacc cgcagttcga aaaataa 807

<210> 6
<211> 804
<212> DNA
<213> Artificial Sequence
<220>
<223> Description of Artificial Sequence: single chain antibody
<400> 6
   cagctgcagc agtctggacc tgagttggtg aagcctgggg cttcagtgaa gatatcctgc 60
   aagacttctg gatacacgtt cactgaatac accatgcact gggtgaaaca gagccatgga 120
   aagagccttg agtggattgg aggtattcat cctaacaatg gtgatactag ctacaaccag 180
   aggttcaagg gaaaggccac attgactgta gacaagtcat ccagcacagc ctacatggag 240
   ctccgcagcc taacatctga ggattctgca gtctattact gtgcaagatg gacgggggac 300
   tttgactact ggggccaagg caccactctc acagtgtcga caggtggagg cggttcaggc 360
   ggaggtggct ctggcggtgg cggaagtgca ctcgacattg tgatgacaca gtctccaaaa 420
   ttcatgtcca catcagtagg agacagggtc agcgtcacct gcaaggccag tcaaaatgtg 480
   ggtactaatg tagcctggta tcaacagaaa ccagggcaat ctcctaaagc actgatttac 540
   tcggcatcct accggtacag tggagtccct gatcgcttca caggcagtgg atctgggaca 600
   gatttcactc tcaccatcag caatgtgcag tctgaagact tggcagagta tttctgtcag 660
   caatataaca gctatccgta cacgttcgga ggggggacca agctggaaat aaaagcggcc 720
   gcaggtgcgc cggtgccgta tccagatccg ctggaaccgc gtggggccgc aagcgcttgg 780
   agccacccgc agttcgaaaa ataa 804

<210> 7
<211> 3918
<212> DNA
<213> Artificial Sequence
<220>
<223> Description of Artificial Sequence: Vector
<400> 7
   ccagcaacgc ggccttttta cggttcctgg ccttttgctg gccttttgct cacatgttct 60
   ttcctgcgtt atcccctgat tctgtggata accgtattac cgcctttgag tgagctgata 120
   ccgctcgccg cagccgaacg accgagcgca gcgagtcagt gagcgaggaa gcggaagagc 180
   gcccaatacg caaaccgcct ctccccgcgc gttggccgat tcattaatgc agctggcacg 240
   acaggtttcc cgactggaaa gcgggcagtg agcgcaacgc aattaatgtg agttagctca 300
   ctcattaggc accccaggct ttacacttta tgcttccggc tcgtatgttg tgtggaattg 360
   tgagcggata acaatttcac acaggaaaca gctatgacca tgattacgcc aagctttgga 420
   gccttttttt tggagatttt caacgtgaaa aaattattat tcgcaattcc tttagttgtt 480
   cctttctatg cggcccagcc ggccatggcc caggtccagt cgacaggtgg aggcggttca 540
   ggcggaggtg gctctggcgg tggcggaagt gcactcatca aacggcggcc gcaggtgcgc 600
   cggtgccgta tccggatccg ctggaaccgc gtgccgcata ggctggcggc ggctctggtg 660
   gtggttctgg tggcggctct gagggtggcg gctctgaggg tggcggttct gagggtggcg 720
   gctctgaggg tggcggttcc ggtggcggct ccggttccgg tgattttgat tatgaaaaaa 780
   tggcaaacgc taataagggg gctatgaccg aaaatgccga tgaaaacgcg ctacagtctg 840
   acgctaaagg caaacttgat tctgtcgcta ctgattacgg tgctgctatc gatggtttca 900
   ttggtgacgt ttccggcctt gctaatggta atggtgctac tggtgatttt gctggctcta 960
   attcccaaat ggctcaagtc ggtgacggtg ataattcacc tttaatgaat aatttccgtc 1020
   aatatttacc ttctttgcct cagtcggttg aatgtcgccc ttatgtcttt ggcgctggta 1080
   aaccatatga attttctatt gattgtgaca aaataaactt attccgtggt gtctttgcgt 1140
   ttcttttata tgttgccacc tttatgtatg tattttcgac gtttgctaac atactgcgta 1200
   ataaggagtc ttaataagaa ttcactggcc gtcgttttac aacgtcgtga ctgggaaaac 1260
   cctggcgtta cccaacttaa tcgccttgca gcacatcccc ctttcgccag ctggcgtaat 1320
   agcgaagagg cccgcaccga tcgcccttcc caacagttgc gcagcctgaa tggcgaatgg 1380
   cgcctgatgc ggtattttct ccttacgcat ctgtgcggta tttcacaccg catacgtcaa 1440
   agcaaccata gtacgcgccc tgtagcggcg cattaagccc ggcgggtgtg gtggttacgc 1500
   gcagcgtgac cgctacactt gccagcgccc tagcccccgc tcctttcgct ttcttccctt 1560
   cctttctcgc cacgttcgcc ggctttcccc gtcaagctct aaatcggggg ctccctttag 1620
   ggttccgatt tagtgcttta cggcacctcg accccaaaaa acttgatttg ggtgatggtt 1680
   cacgtagtgg gccatcgccc tgatagacgg tttttcgtcc tttgacgttc gagtccacgt 1740
   tctttaatag tggactcttg ttccaaactg gaacaatact caaccctatc tcgggctatt 1800
   cttttgattt ataagggatt ttgccgattt cggcctattg gttaaaaaat gagctgattt 1860
   aacaaaaatt taacgcgaat tttaacaaaa tattaacgtt tacaatttta tggtgcagtc 1920
   tcagtacaat ctgctctgat gccgcatagt taagccagcc ccgacacccg ccaacacccg 1980
   ctgacgcgcc ctgacgggct tgtctgctcc cggcatccgc ttacagacaa gctgtgaccg 2040
   tctccgggag ctgcatgtgt cagaggtttt caccgtcatc accgaaacgc gcgagacgaa 2100
   agggcctcgt gatacgccta tttttatagg ttaatgtcat gataataatg gtttcttaga 2160
   cgtcaggtgg cacttttcgg ggaaatgtgc gcggaacccc tatttgttta tttttctaaa 2220
   tacattcaaa tatgtatccg ctcatgagac aataaccctg ataaatgctt caataatatt 2280
   gaaaaaggaa gagtatgagt attcaacatt tccgtgtcgc ccttattccc ttttttgcgg 2340
   cattttgcct tcctgttttt gctcacccag aaacgctggt gaaagtaaaa gatgctgaag 2400
   atcagttggg tgctcgagtg ggttacatcg aactggatct caacagcggt aagatccttg 2460
   agagttttcg ccccgaagaa cgttttccaa tgatgagcac ttttaaagtt ctgctatgtg 2520
   gcgcggtatt atcccgtatt gacgccgggc aagagcaact cggtcgccgc atacactatt 2580
   ctcagaatga cttggttgag tactcaccag tcacagaaaa gcatcttacg gatggcatga 2640
   cagtaagaga attatgcagt gctgccataa ccatgagtga taacactgcg gccaacttac 2700
   ttctgacaac gatcggagga ccgaaggagc taaccgcttt tttgcacaac atgggggatc 2760
   atgtaactcg ccttgatcgt tgggaaccgg agctgaatga agccatacca aacgacgagc 2820
   gtgacaccac gatgcctgta gcaatggcaa caacgttgcg caaactatta actggcgaac 2880
   tacttactct agcttcccgg caacaattaa tagactggat ggaggcggat aaagttgcag 2940
   gaccacttct gcgctcggcc cttccggctg gctggtttat tgctgataaa tctggagccg 3000
   gtgagcgtgg gtctcgcggt atcattgcag cactggggcc agatggtaag ccctcccgta 3060
   tcgtagttat ctacacgacg gggagtcagg caactatgga tgaacgaaat agacagatcg 3120
   ctgagatagg tgcctcactg attaagcatt ggtaactgtc agaccaagtt tactcatata 3180
   tactttagat tgatttaaaa cttcattttt aatttaaaag gatctaggtg aagatccttt 3240
   ttgataatct catgaccaaa atcccttaac gtgagttttc gttccactga gcgtcagacc 3300
   ccgtagaaaa gatcaaagga tcttcttgag atcctttttt tctgcgcgta atctgctgct 3360
   tgcaaacaaa aaaaccaccg ctaccagcgg tggtttgttt gccggatcaa gagctaccaa 3420
   ctctttttcc gaaggtaact ggcttcagca gagcgcagat accaaatact gtccttctag 3480
   tgtagccgta gttaggccac cacttcaaga actctgtagc accgcctaca tacctcgctc 3540
   tgctaatcct gttaccagtg gctgctgcca gtggcgataa gtcgtgtctt accgggttgg 3600
   actcaagacg atagttaccg gataaggcgc agcggtcggg ctgaacgggg ggttcgtgca 3660
   tacagcccag cttggagcga acgacctaca ccgaactgag atacctacag cgtgagctat 3720
   gagaaagcgc cacgcttccc gaagggagaa aggcggacag gtatccggta agcggcaggg 3780
   tcggaacagg agagcgcacg agggagcttc cagggggaaa cgcctggtat ctttatagtc 3840
   ctgtcgggtt tcgccacctc tgacttgagc gtcgattttt gtgatgctcg tcaggggggc 3900
   ggagcctatg gaaaaacg 3918

<210> 8

<211> 3351

<212> DNA

<213> Artificial Sequence

<220>

<223> Description of Artificial Sequence: Vector

<400> 8

gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgct cgagtgggtt acatcgaact ggatctcaac agcggtaaga 360

tccttgagag ttttcgcccc gaagaacgtt ttccaatgat gagcactttt aaagttctgc 420

tatgtggcgc ggtattatcc cgtattgacg ccgggcaaga gcaactcggt cgccgcatac 480

actattctca gaatgacttg gttgagtact caccagtcac agaaaagcat cttacggatg 540

gcatgacagt aagagaatta tgcagtgctg ccataaccat gagtgataac actgcggcca 600

acttacttct gacaacgatc ggaggaccga aggagctaac cgcttttttg cacaacatgg 660

gggatcatgt aactcgcctt gatcgttggg aaccggagct gaatgaagcc ataccaaacg 720

acgagcgtga caccacgatg cctgtagcaa tggcaacaac gttgcgcaaa ctattaactg 780

gcgaactact tactctagct tcccggcaac aattaataga ctggatggag gcggataaag 840

ttgcaggacc acttctgcgc tcggcccttc cggctggctg gtttattgct gataaatctg 900

gagccggtga gcgtgggtct cgcggtatca ttgcagcact ggggccagat ggtaagccct 960

cccgtatcgt agttatctac acgacgggga gtcaggcaac tatggatgaa cgaaatagac 1020

agatcgctga gataggtgcc tcactgatta agcattggta actgtcagac caagtttact 1080

catatatact ttagattgat ttaaaacttc atttttaatt taaaaggatc taggtgaaga 1140

tcctttttga taatctcatg accaaaatcc cttaacgtga gttttcgttc cactgagcgt 1200

cagaccccgt agaaaagatc aaaggatctt cttgagatcc tttttttctg cgcgtaatct 1260

gctgcttgca aacaaaaaaa ccaccgctac cagcggtggt ttgtttgccg gatcaagagc 1320

taccaactct ttttccgaag gtaactggct tcagcagagc gcagatacca aatactgtcc 1380

ttctagtgta gccgtagtta ggccaccact tcaagaactc tgtagcaccg cctacatacc 1440

tcgctctgct aatcctgtta ccagtggctg ctgccagtgg cgataagtcg tgtcttaccg 1500

ggttggactc aagacgatag ttaccggata aggcgcagcg gtcgggctga acggggggtt 1560

cgtgcataca gcccagcttg gagcgaacga cctacaccga actgagatac ctacagcgtg 1620

agctatgaga aagcgccacg cttcccgaag ggagaaaggc ggacaggtat ccggtaagcg 1680

gcagggtcgg aacaggagag cgcacgaggg agcttccagg gggaaacgcc tggtatcttt 1740

atagtcctgt cgggtttcgc cacctctgac ttgagcgtcg atttttgtga tgctcgtcag 1800

gggggcggag cctatggaaa aacgccagca acgcggcctt tttacggttc ctggcctttt 1860

gctggccttt tgctcacatg ttctttcctg cgttatcccc tgattctgtg gataaccgta 1920

ttaccgcctt tgagtgagct gataccgctc gccgcagccg aacgaccgag cgcagcgagt 1980
cagtgagcga ggaagcggaa gagcgcccaa tacgcaaacc gcctctcccc gcgcgttggc 2040
cgattcatta atgcagctgg cacgacaggt ttcccgactg gaaagcgggc agtgagcgca 2100
acgcaattaa tgtgagttag ctcactcatt aggcacccca ggctttacac tttatgcttc 2160
cggctcgtat gttgtgtgga attgtgagcg gataacaatt tcacacagga aacagctatg 2220
accatgatta cgccaagctt gcatgcaaat tctatttcaa ggagacagtc ataatgaaat 2280
acctattgcc tacggcagcc gctggattgt tattactcgc ggcccagccg gccatggccc 2340
aggtgcagct gcaggtcggc ctcgagatca aacgggcggc cgcaggtgcg ccggtgccgt 2400
atccagatcc gctggaaccg cgtggggccg caagcgcttg gagccacccg cagttcgaaa 2460
aataataagg atccgaattc actggccgtc gttttacaac gtcgtgactg ggaaaaccct 2520
ggcgttaccc aacttaatcg ccttgcagca catccccctt tcgccagctg gcgtaatagc 2580
gaagaggccc gcaccgatcg cccttcccaa cagttgcgca gcctgaatgg cgaatggcgc 2640
ctgatgcggt attttctcct tacgcatctg tgcggtattt cacaccgcat acgtcaaagc 2700
aaccatagta cgcgccctgt agcggcgcat taagcccggc gggtgtggtg gttacgcgca 2760
gcgtgaccgc tacacttgcc agcgccctag cccccgctcc tttcgctttc ttcccttcct 2820
ttctcgccac gttcgccggc tttccccgtc aagctctaaa tcgggggctc cctttagggt 2880
tccgatttag tgctttacgg cacctcgacc ccaaaaaact tgatttgggt gatggttcac 2940
gtagtgggcc atcgccctga tagacggttt ttcgcccttt gacgttcgag tccacgttct 3000
ttaatagtgg actcttgttc caaactggaa caatactcaa ccctatctcg ggctattctt 3060
ttgatttata agggattttg ccgatttcgg cctattggtt aaaaaatgag ctgatttaac 3120
aaaaatttaa cgcgaatttt aacaaaatat taacgtttac aattttatgg tgcagtctca 3180
gtacaatctg ctctgatgcc gcatagttaa gccagccccg acacccgcca acacccgctg 3240
acgcgccctg acgggcttgt ctgctcccgg catccgctta cagacaagct gtgaccgtct 3300
ccgggagctg catgtgtcag aggttttcac cgtcatcacc gaaacgcgcg a 3351

<210> 9
<211> 23
<212> DNA
<213> Artificial Sequence
<220>
<223> Description of Artificial Sequence: primer
<400> 9
gaggtgcagc ttcaggagtc agg 23

<210> 10
<211> 23
<212> DNA
<213> Artificial Sequence
<220>

<223> Description of Artificial Sequence: primer

<400> 10

caggtgcagc tgaaggagtc agg                                              23

<210> 11

<211> 23

<212> DNA

<213> Artificial Sequence

<220>

<223> Description of Artificial Sequence: primer

<400> 11

gaggtccagc tgcaacagtc tgg                                              23

<210> 12

<211> 23

<212> DNA

<213> Artificial Sequence

<220>

<223> Description of Artificial Sequence: primer

<400> 12

gaggttcagc tgcagcagtc tgg                                              23

<210> 13

<211> 23

<212> DNA

<213> Artificial Sequence

<220>

<223> Description of Artificial Sequence: primer

<400> 13

caggtccaac tgcagcagcc tgg                                              23

<210> 14

<211> 23

<212> DNA

<213> Artificial Sequence

<220>

<223> Description of Artificial Sequence: primer

<400> 14

caggttcagc tgcagcagtc tgg                                              23

<210> 15
<211> 23
<212> DNA
<213> Artificial Sequence
<220>
<223> Description of Artificial Sequence: primer
<400> 15
gaggtgaagc tggtggagtc tgg                                                23

<210> 16
<211> 23
<212> DNA
<213> Artificial Sequence
<220>
<223> Description of Artificial Sequence: primer
<400> 16
gaggtgaagc tggtggaatc tgg                                                23

<210> 17
<211> 23
<212> DNA
<213> Artificial Sequence
<220>
<223> Description of Artificial Sequence: primer
<400> 17
gaggttcagc ttcagcagtc tgg                                                23

<210> 18
<211> 24
<212> DNA
<213> Artificial Sequence
<220>
<223> Description of Artificial Sequence: primer
<400> 18
tgaggagacg gtgaccgtgg tccc                                               24

<210> 19
<211> 24

<212> DNA
<213> Artificial Sequence
<220>
<223> Description of Artificial Sequence: primer
<400> 19
tgaggagact gtgagagtgg tgcc                                              24

<210> 20
<211> 24
<212> DNA
<213> Artificial Sequence
<220>
<223> Description of Artificial Sequence: primer
<400> 20
tgcagagaca gtgaccagag tccc                                              24

<210> 21
<211> 24
<212> DNA
<213> Artificial Sequence
<220>
<223> Description of Artificial Sequence: primer
<400> 21
tgaggagacg gtgactgagg ttcc                                              24

<210> 22
<211> 23
<212> DNA
<213> Artificial Sequence
<220>
<223> Description of Artificial Sequence: primer
<400> 22
gacattgtga tgacacagtc tcc                                               23

<210> 23
<211> 23
<212> DNA
<213> Artificial Sequence
<220>

<223> Description of Artificial Sequence: primer
<400> 23
gatgttgtga tgacccaaac tcc                                              23

<210> 24
<211> 23
<212> DNA
<213> Artificial Sequence
<220>
<223> Description of Artificial Sequence: primer
<400> 24
gatatccaga tgacacagac tcc                                              23

<210> 25
<211> 23
<212> DNA
<213> Artificial Sequence
<220>
<223> Description of Artificial Sequence: primer
<400> 25
caaattgttc tcacccagtc tcc                                              23

<210> 26
<211> 23
<212> DNA
<213> Artificial Sequence
<220>
<223> Description of Artificial Sequence: primer
<400> 26
caggctgttg tgactcagga atc                                              23

<210> 27
<211> 24
<212> DNA
<213> Artificial Sequence
<220>
<223> Description of Artificial Sequence: primer
<400> 27
tttgatttcc agcttggtgc ctcc                                             24

<210> 28

<211> 24

<212> DNA

<213> Artificial Sequence

<220>

<223> Description of Artificial Sequence: primer

<400> 28

ttttatttcc agcttggtcc cccc                                              24

<210> 29

<211> 24

<212> DNA

<213> Artificial Sequence

<220>

<223> Description of Artificial Sequence: primer

<400> 29

tttcagctcc agcttggtcc cagc                                              24

<210> 30

<211> 24

<212> DNA

<213> Artificial Sequence

<220>

<223> Description of Artificial Sequence: primer

<400> 30

acctaggaca gtgaccttgg ttcc                                              24

<210> 31

<211> 56

<212> DNA

<213> Artificial Sequence

<220>

<223> Description of Artificial Sequence: primer

<400> 31

gtcctcgcaa ctgcggccca gccggccatg gccgaggtgc agcttcagga gtcagg      56

<210> 32

<211> 56

<212> DNA
<213> Artificial Sequence
<220>
<223> Description of Artificial Sequence: primer
<400> 32
gtcctcgcaa ctgcggccca gccggccatg gcccaggtgc agctgaagga gtcagg     56

<210> 33
<211> 56
<212> DNA
<213> Artificial Sequence
<220>
<223> Description of Artificial Sequence: primer
<400> 33
gtcctcgcaa ctgcggccca gccggccatg gccgaggtcc agctgcaaca gtctgg     56

<210> 34
<211> 56
<212> DNA
<213> Artificial Sequence
<220>
<223> Description of Artificial Sequence: primer
<400> 34
gtcctcgcaa ctgcggccca gccggccatg gccgaggttc agctgcagca gtctgg     56

<210> 35
<211> 56
<212> DNA
<213> Artificial Sequence
<220>
<223> Description of Artificial Sequence: primer
<400> 35
gtcctcgcaa ctgcggccca gccggccatg gcccaggtcc aactgcagca gcctgg     56

<210> 36
<211> 56
<212> DNA
<213> Artificial Sequence
<220>

<223> Description of Artificial Sequence: primer

<400> 36

gtcctcgcaa ctgcggccca gccggccatg gcccaggttc agctgcagca gtctgg     56

<210> 37

<211> 56

<212> DNA

<213> Artificial Sequence

<220>

<223> Description of Artificial Sequence: primer

<400> 37

gtcctcgcaa ctgcggccca gccggccatg gccgaggtga agctggtgga gtctgg     56

<210> 38

<211> 56

<212> DNA

<213> Artificial Sequence

<220>

<223> Description of Artificial Sequence: primer

<400> 38

gtcctcgcaa ctgcggccca gccggccatg gccgaggtga agctggtgga atctgg     56

<210> 39

<211> 56

<212> DNA

<213> Artificial Sequence

<220>

<223> Description of Artificial Sequence: primer

<400> 39

gtcctcgcaa ctgcggccca gccggccatg gccgaggttc agcttcagca gtccgg     56

<210> 40

<211> 36

<212> DNA

<213> Artificial Sequence

<220>

<223> Description of Artificial Sequence: primer

<400> 40

gagtcattct cgtgtcgaca cggtgaccgt ggtccc                           36

<210> 41
<211> 36
<212> DNA
<213> Artificial Sequence
<220>
<223> Description of Artificial Sequence: primer
<400> 41
gagtcattct cgtgtcgaca ctgtgagagt ggtgcc                                36

<210> 42
<211> 36
<212> DNA
<213> Artificial Sequence
<220>
<223> Description of Artificial Sequence: primer
<400> 42
gagtcattct cgtgtcgaca cagtgaccag agtccc                                36

<210> 43
<211> 36
<212> DNA
<213> Artificial Sequence
<220>
<223> Description of Artificial Sequence: primer
<400> 43
gagtcattct cgtgtcgaca cggtgactga ggttcc                                36

<210> 44
<211> 41
<212> DNA
<213> Artificial Sequence
<220>
<223> Description of Artificial Sequence: primer
<400> 44
tgagcacaca gtgcactcga cattgtgatg acacagtctc c                          41

<210> 45
<211> 41

<212> DNA
<213> Artificial Sequence
<220>
<223> Description of Artificial Sequence: primer
<400> 45
tgagcacaca gtgcactcga tgttgtgatg acccaaactc c                41

<210> 46
<211> 41
<212> DNA
<213> Artificial Sequence
<220>
<223> Description of Artificial Sequence: primer
<400> 46
tgagcacaca gtgcactcga tatccagatg acacagactc c                41

<210> 47
<211> 41
<212> DNA
<213> Artificial Sequence
<220>
<223> Description of Artificial Sequence: primer
<400> 47
tgagcacaca gtgcactcca aattgttctc acccagtctc c                41

<210> 48
<211> 41
<212> DNA
<213> Artificial Sequence
<220>
<223> Description of Artificial Sequence: primer
<400> 48
tgagcacaca gtgcactcca ggctgttgtg actcaggaat c                41

<210> 49
<211> 48
<212> DNA
<213> Artificial Sequence
<220>

<223> Description of Artificial Sequence: primer

<400> 49

gagtcattct cgacttgcgg ccgctttgat ttccagcttg gtgcctcc 48

<210> 50

<211> 48

<212> DNA

<213> Artificial Sequence

<220>

<223> Description of Artificial Sequence: primer

<400> 50

gagtcattct cgacttgcgg ccgcttttat ttccagcttg gtcccccc 48

<210> 51

<211> 48

<212> DNA

<213> Artificial Sequence

<220>

<223> Description of Artificial Sequence: primer

<400> 51

gagtcattct cgacttgcgg ccgctttcag ctccagcttg gtcccagc 48

<210> 52

<211> 48

<212> DNA

<213> Artificial Sequence

<220>

<223> Description of Artificial Sequence: primer

<400> 52

gagtcattct cgacttgcgg ccgcacctag gacagtgacc ttggttcc 48

<210> 53

<211> 17

<212> DNA

<213> Artificial Sequence

<220>

<223> Description of Artificial Sequence: primer

<400> 53

ccccacgcgg ttccagc 17

<210> 54
<211> 17
<212> DNA
<213> Artificial Sequence
<220>
<223> Description of Artificial Sequence: primer
<400> 54
tacctattgc ctacggc                                                    17

<210> 55
<211> 21
<212> DNA
<213> Artificial Sequence
<220>
<223> Description of Combined DNA/RNA Molecule: ribonucleotide
<220>
<223> Description of Artificial Sequence: primer
<400> 55
ccagtcactt gtctggagct t                                               21

<210> 56
<211> 21
<212> DNA
<213> Artificial Sequence
<220>
<223> Description of Combined DNA/RNA Molecule: ribonucleotide
<220>
<223> Description of Artificial Sequence: primer
<400> 56
gagcttgaag aagtgggtat t                                               21

<210> 57
<211> 21
<212> DNA
<213> Artificial Sequence
<220>
<223> Description of Combined DNA/RNA Molecule: ribonucleotide
<220>

<223> Description of Artificial Sequence: primer

<400> 57

tgctggtggc attactggtg c                                                21

<210> 58

<211> 23

<212> DNA

<213> Artificial Sequence

<220>

<223> Description of Combined DNA/RNA Molecule: ribonucleotide

<220>

<223> Description of Artificial Sequence: primer

<400> 58

gctgtcctgg ccaccccgtg gaa                                              23

<210> 59

<211> 22

<212> DNA

<213> Artificial Sequence

<220>

<223> Description of Combined DNA/RNA Molecule: ribonucleotide

<220>

<223> Description of Artificial Sequence: primer

<400> 59

ggcgcggagc tgcggaatgc ct                                               22

<210> 60

<211> 22

<212> DNA

<213> Artificial Sequence

<220>

<223> Description of Combined DNA/RNA Molecule: ribonucleotide

<220>

<223> Description of Artificial Sequence: primer

<400> 60

cctcgctgag gatgttcggg tt                                               22

<210> 61

<211> 21

<212> DNA
<213> Artificial Sequence
<220>
<223> Description of Combined DNA/RNA Molecule: ribonucleotide
<220>
<223> Description of Artificial Sequence: primer
<400> 61
cccgtgaaca cagctgaggt t                                                21

<210> 62
<211> 23
<212> DNA
<213> Artificial Sequence
<220>
<223> Description of Combined DNA/RNA Molecule: ribonucleotide
<220>
<223> Description of Artificial Sequence: primer
<400> 62
caaggtggac ccacgagagc ttt                                              23

<210> 63
<211> 30
<212> DNA
<213> Artificial Sequence
<220>
<223> Description of Artificial Sequence: primer
<400> 63
cttacaacca caatccctgg gcacaatttt                                       30

<210> 64
<211> 24
<212> DNA
<213> Artificial Sequence
<220>
<223> Description of Artificial Sequence: primer
<400> 64
ctttgtgggc cctctgggct caat                                             24

<210> 65

<211> 24
<212> DNA
<213> Artificial Sequence
<220>
<223> Description of Artificial Sequence: primer
<400> 65
tgaaatgggc ccgctgggct caag                                              24

<210> 66
<211> 24
<212> DNA
<213> Artificial Sequence
<220>
<223> Description of Artificial Sequence: primer
<400> 66
gggcttgggt attctaggct cgat                                              24

<210> 67
<211> 19
<212> DNA
<213> Artificial Sequence
<220>
<223> Description of Artificial Sequence: primer
<400> 67
tcagaggaag gaaacaggt                                                    19

<210> 68
<211> 20
<212> DNA
<213> Artificial Sequence
<220>
<223> Description of Artificial Sequence: primer
<400> 68
ctggatggtg ggaagatgga                                                   20

<210> 69
<211> 268
<212> PRT
<213> Artificial Sequence

<220>
<223> Description of Artificial Sequence: single chain antibody
<400> 69
Gln Val Gln Leu Gln Gln Pro Gly Ala Val Leu Val Arg Pro Gly Ala
1               5                   10                  15
Ser Val Lys Leu Ser Cys Lys Ala Leu Gly Tyr Thr Phe Thr Asp His
            20                  25                  30
Glu Met His Trp Val Arg Gln Thr Pro Val His Gly Leu Glu Trp Ile
        35                  40                  45
Gly Ala Ile His Pro Gly Ser Gly Val Thr Ala Tyr Asn Gln Lys Phe
    50                  55                  60
Lys Gly Lys Ala Thr Leu Thr Ala Asp Lys Ser Ser Ser Thr Ala Tyr
65                  70                  75                  80
Met Glu Leu Ser Ile Leu Thr Ser Glu Asp Ser Ala Val Tyr Tyr Cys
                85                  90                  95
Thr Pro Leu Trp Leu Arg Arg Asp Trp Gly Gln Gly Thr Thr Leu Thr
            100                 105                 110
Val Ser Thr Gly Gly Gly Gly Ser Gly Gly Gly Gly Ser Gly Gly Gly
        115                 120                 125
Gly Ser Ala Leu Asp Val Val Met Thr Gln Thr Pro Lys Phe Met Ser
    130                 135                 140
Thr Ser Val Gly Asp Arg Val Ser Val Thr Cys Lys Ala Ser Gln Asn
145                 150                 155                 160
Val Gly Thr Asn Val Ala Trp Tyr Gln Gln Lys Pro Gly Gln Ser Pro
                165                 170                 175
Lys Ala Leu Ile Tyr Ser Ala Ser Tyr Arg Tyr Ser Gly Val Pro Asp
            180                 185                 190
Arg Phe Thr Gly Ser Gly Ser Gly Thr Asp Phe Thr Leu Thr Ile Ser
        195                 200                 205
Asn Val Gln Ser Glu Asp Leu Ala Asp Tyr Phe Cys Gln Gln Tyr Asn
    210                 215                 220
Thr Tyr Pro Leu Thr Phe Gly Ala Gly Thr Lys Leu Glu Leu Lys Ala
225                 230                 235                 240
Ala Ala Gly Ala Pro Val Pro Tyr Pro Asp Pro Leu Glu Pro Arg Gly
                245                 250                 255
Ala Ala Ser Ala Trp Ser His Pro Gln Phe Glu Lys
            260                 265

<210> 70

<211> 268
<212> PRT
<213> Artificial Sequence
<220>
<223> Description of Artificial Sequence: single chain antibody
<400> 70
Glu Val Gln Leu Gln Gln Ser Gly Ala Glu Leu Val Arg Pro Gly Ala
1               5                   10                  15
Ser Val Lys Leu Ser Cys Lys Ala Leu Gly Tyr Thr Phe Thr Asp His
            20                  25                  30
Glu Met His Trp Val Arg Gln Thr Pro Val His Gly Leu Glu Trp Ile
        35                  40                  45
Gly Ala Ile His Pro Gly Ser Gly Val Thr Ala Tyr Asn Gln Lys Phe
    50                  55                  60
Lys Gly Lys Ala Thr Leu Thr Ala Asp Lys Ser Ser Ser Thr Ala Tyr
65                  70                  75                  80
Met Glu Leu Ser Ile Leu Thr Ser Glu Asp Ser Ala Val Tyr Tyr Cys
                85                  90                  95
Thr Pro Leu Trp Leu Arg Arg Asp Trp Gly Gln Gly Thr Thr Leu Thr
            100                 105                 110
Val Ser Thr Gly Gly Gly Gly Ser Gly Gly Gly Gly Ser Gly Gly Gly
        115                 120                 125
Gly Ser Ala Leu Asp Ile Val Met Thr Gln Ser Pro Lys Phe Met Ser
    130                 135                 140
Thr Ser Val Gly Asp Arg Val Ser Val Thr Cys Lys Ala Ser Gln Asn
145                 150                 155                 160
Val Ala Thr Asn Val Val Trp Tyr Gln Gln Lys Ser Gly Gln Ser Pro
                165                 170                 175
Lys Ala Leu Ile Tyr Ser Ala Ser Tyr Arg Tyr Ser Gly Val Pro Asp
            180                 185                 190
Arg Phe Thr Gly Ser Gly Ser Gly Thr Asp Phe Thr Leu Thr Ile Ser
        195                 200                 205
Asn Val Gln Ser Glu Asp Leu Ala Glu Tyr Phe Cys Gln Gln Tyr Asn
    210                 215                 220
Ser Tyr Pro Leu Thr Phe Gly Ala Gly Thr Lys Leu Glu Leu Lys Ala
225                 230                 235                 240
Ala Ala Gly Ala Pro Val Pro Tyr Pro Asp Pro Leu Glu Pro Arg Gly
                245                 250                 255
Ala Ala Ser Ala Trp Ser His Pro Gln Phe Glu Lys

            260                 265

<210> 71
<211> 807
<212> DNA
<213> Artificial Sequence
<220>
<223> Description of Artificial Sequence: single chain antibody
<400> 71
caggtccaac tgcagcagcc tggggctgta ctggtgaggc ctggggcttc agtgaagctg 60
tcctgcaagg ctttgggcta cacatttact gaccatgaaa tgcactgggt gaggcagaca 120
cctgtacatg gcctggaatg gattggagct attcatccag gaagtggtgt tactgcctac 180
aatcagaagt tcaagggcaa ggccacactg actgcagaca aatcctccag cacagcctac 240
atggagctca gtattctgac atctgaagac tctgctgtct attactgtac accactgtgg 300
ttacgacggg actggggcca aggcaccacc ctcacagtgt cgacaggtgg aggcggttca 360
ggcggaggtg gctctggcgg tggcggaagt gcactcgatg ttgtgatgac ccaaactcca 420
aaattcatgt ccacatcagt aggagacagg gtcagcgtca cctgcaaggc cagtcagaat 480
gtgggtacta atgtagcctg gtatcaacag aaaccagggc aatctcctaa agcactgatt 540
tactcggcat cctaccggta cagtggagtc cctgatcgct tcacaggcag tggatctggg 600
acagatttca ctctcaccat cagcaatgtg cagtctgaag acttggcaga ctatttctgt 660
cagcaatata acacctatcc tctcacgttc ggtgctggga ccaagctgga gctgaaagcg 720
gccgcaggtg cgccggtgcc gtatccagat ccgctggaac cgcgtggggc cgcaagcgct 780
tggagccacc cgcagttcga aaaataa 807

<210> 72
<211> 807
<212> DNA
<213> Artificial Sequence
<220>
<223> Description of Artificial Sequence: single chain antibody
<400> 72
gaggttcagc tgcagcagtc tggggctgag ctggtgaggc ctggggcttc agtgaagctg 60
tcctgcaagg ctttgggcta cacatttact gaccatgaaa tgcactgggt gaggcagaca 120
cctgtacatg gcctggaatg gattggagct attcatccag gaagtggtgt tactgcctac 180
aatcagaagt tcaagggcaa ggccacactg actgcagaca aatcctccag cacagcctac 240
atggagctca gtattctgac atctgaagac tctgctgtct attactgtac accactgtgg 300
ttacgacggg actggggcca aggcaccact ctcacagtgt cgacaggtgg aggcggttca 360
ggcggaggtg gctctggcgg tggcggaagt gcactcgaca ttgtgatgac acagtctcca 420
aaattcatgt ccacatcagt aggagacagg gtcagcgtca cctgcaaggc cagtcagaat 480

gtggctacta atgtagtctg gtatcaacag aaatcagggc aatctcctaa agcactgatt 540
tactcggcat cctaccggta cagtggagtc cctgatcgct tcacaggcag tggatctggg 600
acagatttca ctctcaccat cagcaatgtg cagtctgaag acttggcaga gtatttctgt 660
cagcaatata acagctatcc tctcacgttc ggtgctggga ccaagctgga gctgaaagcg 720
gccgcaggtg cgccggtgcc gtatccagat ccgctggaac cgcgtggggc cgcaagcgct 780
tggagccacc cgcagttcga aaaataa 807

## Description

### BACKGROUND OF THE INVENTION

Malignant tumors shed cells, which migrate to new tissues and create secondary tumors. The process of generating secondary tumors is called metastasis and is a complex process in which tumor cells colonize sites distant from the primary tumor. Liotta ((1986) Cancer Res. 46, 1-7) has proposed a three-step hypothesis for the process of metastasis: The first step is tumor cell attachment via cell surface receptors. The anchored tumor cell next secretes hydrolytic enzymes or induces host cells to secrete enzymes, which can degrade the matrix locally. Matrix lysis most likely takes place in a highly localized region close to the tumor cell surface. The third step is tumor cell locomotion into the region of the matrix modified by proteolysis. Recently, research has been focused on identifying specific proteins involved in metastasis, which can be used as a basis for better diagnostic or improved therapeutic strategies. Cell adhesion molecules (CAM's), which mediate cell - cell or cell - matrix interactions, have been proposed to be involved in the process of metastasis. Cell adhesion in normal cells involves interactions between numerous cell surface proteins. Specifically, adhesive interactions are known to involve interactions between substances surrounding the cell and extracellular adhesion receptors. It has also become apparent that cell adhesion molecules fulfill much more complex functions which may result in cells acquiring the ability to proliferate and invade host tissues. Cell adhesion receptors comprise molecules such as selectins, cadherins, integrins and immunoglobulins.

The integrin receptor family has been studied intensively and it is well-known that the adhesion receptor alpha v beta 3 integrin contributes to tumor cell functions and is potentially involved in melanoma growth and metastasis. Felding-Habermann et al (Clin Exp Metastasis (2002) 19, 427-36) showed that expression of integrin alpha v beta 3 promotes the metastatic phenotype in human melanoma by supporting specific adhesive, invasive and migratory properties of the tumor cells. Inhibition of alpha v beta 3 expressed on melanoma cells reduced metastasis significantly and further prolonged animal survival. Haier et al showed that the ability of tumor cells to adhere to extracellular matrix proteins is an important factor in metastasis formation (Haier et al (2002) J. Exp. Ther. Oncol. 2, 237-45 and Haier et al. (1999) Br. J. of Cancer 80, 1867-74).

The process of metastasis formation also depends on the invasiveness of tumor cells. Invasiveness describes the potential of a tumor cell to migrate and disregard the extracellular matrix. The extracellular matrix consists of a supramolecular aggregate of connective tissue proteins including collagens, elastin, fibronectin, laminin, glycoproteins and glycosaminoglycans. It has normally supportive and nutritive function for development and organization of tissue, but also serves as a physical barrier to limit the migration of normal cells. Although considerable information is available on the digestion of isolated connective tissue proteins by tumor enzymes, less is known about the mechanisms by which tumor cells penetrate highly complex mixtures of extracellular matrix proteins.

### OBJECT OF THE INVENTION

It was therefore one object of the invention to identify and isolate agents modulating the adhesion and/or invasion behavior of cancer or metastatic cells preferably via a receptor, which is predominantly expressed on tumor cells.

It was the achievement of the inventors of the present invention to establish an experimental set up including various functional assays to identify and eventually isolate molecules, which have as main characteristic the capability to modulate the adhesion and/or invasion behavior of a cell. In additional experiments the inventor could specify the involved receptor molecule, a poliovirus receptor (PVR), derivative and/or analog thereof, which was involved in the adhesion and invasive behavior of a cell and which was modulated by the molecules according to the present invention.

The PVR is a transmembrane glycoprotein with a N-terminal signal sequence, three extracellular immunoglobulin (Ig)-like domains, a transmembran domain and a cytoplasmic tail. It can be also described as a member of the immunoglobulin superfamily having a molecular size of approximately 80 kDa and a specific structure composed of three Ig-like domains, specifically an outermost V-like domain followed by two C2-like domains. An alternative nomenclature for PVR is CD155.

Originally, PVR was discovered as the cellular receptor for poliovirus, the causative agent for poliomyelitis. The interaction between the poliovirus and PVR was studied in great detail (see e.g. Koike et al. (1991) Proc. Nat. Acad. Sci. USA 88, 4104-08; Belnap et al. (2000) PNAS 97, 73-78; Racaniello (1996) Structure 4, 769-73; Racaniello (1996) Proc. Natl. Acad. Sci. USA 93, 11378-81).

Nonetheless, the physiological function(s) of PVR remained largely unknown and the biological importance of PVR just slowly begins to be elucidated. Solecki et al. ((2002) J. Biol. Chem. 277, 25697-700) recently discussed PVR/CD155 as being involved in mediating cell adhesion to extracellular matrix molecules as well as being up-regulated in neuroectodermal tumors. An analysis of the transcriptional regulation of CD155 revealed two potent regulators (activator protein-2 and nuclear respiratory factor-1) of the CD 155 transcription. Both transcription factors are expressed during CNS development and may direct the developmental CD155 expression. The over-expresssion of CD155 in neuroectodermal tumors suggests the existence of gene regulatory pathways in neuroectodermal tumors, which reactivate CD155 expression.

Mason et al. ((2001) Gut 49, 236-40) revealed that the CD155 gene is also overexpressed in colorectal tumors and showed that the overexpression begins at very early stage in tumorigenesis and continues to late stages.

Lange et al. ((2001) Virol. 285, 218-227) further report a mediation of cell adhesion by the various polio receptor related (PRR) proteins and a specific CD155/vitronectin interaction, which seems to be required for the establishment of a proper immune response inside the germinal centers of secondary lymphoid tissue.

Nonetheless, the physiological function of CD155/PVR is not fully understood. The determination of the physiological role of a protein is a prerequisite for deciding whether interference with this protein's function might be a possible avenue for the treatment of disease or not. It must be kept in mind that in a physiological setting, which means e.g. in a naturally occurring tumor cell of a patient, PVR is overexpressed. Such overexpression may allow a cell to react differently to stimulation, and thereby can modulate and change physiological PVR function.

Accordingly, it was a further object of the present invention to provide means and methods to modulate the functions of PVR and thereby to contribute to a better understanding of the role of PVR.

Additionally, it was a further object of the present invention to develop pharmaceutical compositions or drugs, which inhibit PVR mediated functions such as adhesiveness and/or invasiveness of cells.

### SUMMARY OF THE INVENTION

The present invention provides for the first time molecules, which modulate the PVR functions necessary for adhesion, trafficking, invasion and/or a metastatic potential of cells. These molecules are selected from the group comprising small chemical compounds, oligonucleotides, peptides, oligopeptides, polypeptides, proteins, antibodies, antibody fragments, anti-idiotypic antibodies and/or bioconjugates.

One feature of these molecules is that each of them binds specifically to one or more intra- or extracellular domains of the PVR, CD155 or any derivative or analog thereof, which is expressed on cells being involved in a proliferative disease or disorder, having a metastatic potential or deriving from a naturally occurring tumor.

This specific binding itself and/or the activation or induction of a label, which is attached to the molecule, starts the modulation of the PVR function(s). In other words due to a blocking and/or destruction of an active site in the PVR the mediated function(s), which influence the adhesion, trafficking or invasion behavior of a cell, are either induced, increased, stabilized, strengthened, prevented, inhibited, decreased and/or diminished.

In an unbiased screen for molecules that can inhibit adhesiveness and invasiveness of naturally occurring tumor cells surprisingly specific antibody fragments binding to the extracellular domain of PVR and having the amino acid sequence SEQ ID No.: 3 or SEQ ID No.: 4 has been identified as such a modulator.

The present invention further comprises peptides, polypeptides, proteins, antibodies or antibody fragments comprising the amino acid sequence LWLRRD (SEQ ID No.: 1) and/or WTGDFDY (SEQ ID No.: 2), which can specifically bind to the extracellular domain of PVR and inhibit the adhesion and/or invasive behavior of a cell.

The present invention relates further to the DNA sequences (SEQ ID No.: 5 or 6) translating to SEQ ID No.: 3 or SEQ ID No.: 4, to DNA sequences corresponding or homologous to SEQ ID No.: 5 or 6, but also to the DNA sequences, which have a degenerated code but still translate into the amino acid sequences as above.

In a further embodiment the invention relates to nucleic acid molecules encoding any of the molecules or polypeptides of the invention.

The invention further provides expression vectors encoding any of the DNA sequences as above, as well as to host cells comprising such a vector. Still further the invention provides a pharmaceutical composition, a bioconjugate or a kit comprising any of the amino acid sequences, DNA sequences or vectors as above.

The invention provides the molecules as above to modulate or inhibit PVR function as medicament or drug and/or for the manufacturing of a medicament or drug for the treatment or prevention of adhesion, trafficking, invasion and/or metastatic potential of naturally occurring cancer cells, wherein adhesiveness, invasiveness and/or metastatic potential of said cancer cells depend on PVR mediated function.

In a further embodiment the invention relates to a method for the identification of a ligand useful for inhibiting the adhesiveness or invasiveness of a naturally occurring cancer cell, particularly the identification of such ligands that bind to the extracellular domain of PVR. This method comprises as a first step the establishment of a ligand library. Typical ligands represented in such a library are small chemical compounds, oligonucleotides, peptides, oligopeptides, polypeptides, proteins, antibodies, antibody fragments, anti-idiotypic antibodies and/or bioconjugates. The library is subsequently screened for tumor-specific ligands, which are further tested to identify ligands, which modulate the PVR function(s) in assays further provided by the present invention.

In a further embodiment the invention provides several diagnostic methods to determine adhesiveness and/or invasiveness and, thus, the metastatic potential of a naturally occurring cancer cell comprising the isolation of naturally occurring cancer cells, applying the assay to determine the adhesiveness or invasiveness of the cells and analyzing the results compared to normal cells.

In a further embodiment the invention relates to a method of treating or preventing adhesion, trafficking, invasion and/or metastatic potential of cells in a patient, wherein the method comprises administering to a person in need thereof a therapeutically effective amount of the molecules, the vectors and/or the pharmaceutical composition according to the invention.

### DETAILED DESCRIPTION OF THE INVENTION

In order that the invention described herein may be more fully understood, the following detailed description and definitions are provided.

A solution to the object of the present invention, namely to modulate the adhesion, trafficking and/or invasion behavior of a cell is achieved by the features of the independent claims.

The molecules, which had been identified by the inventors, have a common characteristic as they all bind specifically to the PVR receptor or any of its derivatives. The molecules or ligands specifically binding to the PVR according to the invention bind the PVR or any of its derivatives regardless of the physiological set up, thus on a naturally presented receptor, an isolated receptor as well as on a column-bound receptor.

The term "PVR" as used herein comprises the originally discovered Poliovirus receptor, which correlates with the CD155 molecule. PVR as used herein further comprises any member of the known family of related PVR's, which originate either by alternative RNA splicing or as transcripts of related homologous genes, namely human PRR1, PRR2 and PRR3 gene (Racaniello (1996) Proc. Natl. Acad. Sci. USA, Vol. 93 pp 11378-81; Lange et al. (2001) Virol. 285, 218-227). The abbreviation PVR is used hereinafter even if the whole family of related receptors, like CD155, PRR1 to PRR3, or derivatives, analogs or fragments thereof are addressed.

The molecule according to the invention binds to one or more intra- or extracellular regions or domains of the PVR. The extracellular region of PVR is defined as that part of the PVR protein outside of the cellular membrane, particularly the extracellular amino acid loops between amino acid 26 and amino acid 854. More specifically the V-like domain and the two C2-like domains. Thus, molecules according to the invention specifically recognize one or more epitopes of PVR, or epitopes of conserved variants of PVR as listed above.

The term "epitope" as used herein includes any protein determinant capable of specific binding to an immunoglobulin or an antibody fragment. Epitopic determinants usually consist of chemically active surface groupings of molecules such as exposed amino acids, amino sugars, or other carbohydrate side chains and usually have specific three-dimensional structural characteristics, as well as specific charge characteristics. It should be appreciated that PVR is a glycoprotein, so not only the amino acid sequence, but also the sugar modifications on it are considered as being epitopes or recognisable part of the extracellular region of PVR.

The molecules according to the present invention are characterized by an additional functional definition, namely, that they have all the ability to specifically bind the PVR as a ligand e.g. on a living or fixed cell. A ligand "binding specifically to a PVR" as mentioned herein can be a ligand, which binds to PVR under the buffer conditions given in the Examples. The dissociation constant between the ligand and PVR can be measured, e.g. by use of the so-called BIACORE System (see, for example, Fivash et al. Curr Opin Biotechnol. (1998) 9, 97-101) and "binding specifically" can then be understood to mean that the dissociation constant between the ligand and PVR is lower than 10 µM, preferably lower than 1 µM, more preferably lower than 500, 400, 300, 200, 100, 50, 20 nM, most preferable from 0,1 nM to 20 nM if measured under standard conditions, for example at 20°C, ambient pressure and in a suitable buffer, e.g. 20 mM Tris, 100 mM NaCl, 0,1 mM EDTA at pH of 7.0.

Further, the molecules according to the present invention have the ability to modulate the physiological functions and/or interactions of PVR. A "molecule modulating PVR function", is a molecule resulting in modulation of the biological activity of PVR, which are for example a mediation of adhesiveness to extracellular matrix structures of the cell expressing PVR, a mediation of trafficking through normal adjacent or distant tissue, organs and/or extracellular matrix structures of the cell expressing PVR, and/or a mediation of invasiveness of the cell expressing PVR into normal adjacent or distant tissue, organs or extracellular matrix structures. This modulation of the biological activity of PVR can be assessed by measuring one or more indicators of PVR's biological activity, such as PVR dependent invasiveness or PVR dependent adhesiveness. These indicators of PVR's biological activity can be assessed by in *in vitro* or *in vivo* assays (see Examples). The ability of a molecule to inhibit PVR activity is primarily assessed by inhibition of PVR-induced adhesiveness compared with adhesion of invasive human cancer cells (e.g. sarcoma cells, breast cancer cells).

A molecule modulating PVR function of the invention is not a molecule which is a general inhibitor of protein function, like a protease, like a denaturing agent, e.g. urea or guanidinium hydrochloride, like heavy metal atoms or like small molecules (e.g. aldehydes or isocyanates) reacting covalently and non-specifically with biomolecules (lipids, proteins, sugars). A molecule modulating PVR function is characterized - *inter alia* - by its ability to modulate PVR function at a concentration at which it does not inhibit the function of the insulin receptor (e.g. as determined in an anti-Phosphotyrosine Western Blot Assay, see e.g. B. Cariou et al. (2002) J Biol Chem., 277, 4845-52) or that of the acetylcholin receptor (e.g. as determined by measuring the Ca influx, see M. Montiel et al. (2001) Biochem Pharmacol. 63, 337-42.) or that of the B-CAM cell surface glycoprotein (e.g. by determining binding of hemoglobin A red blood cells (AA RBCs) to immobilized laminin as described in the section "Flow chamber assays" on page 2551 of Udani et al. (1998) J. Clin. Invest. 101, 2550-2558). Only a molecule modulating PVR function but at the same concentration not significantly affecting the function of the other three receptors mentioned is a molecule as used in this patent.

According to one embodiment the molecule modulating the PVR function binds to the PVR, preferably - but not exclusively - to an extracellular part of the PVR, in a way that the whole receptor or at least one or more active domains of the receptor are covered by the molecule and, thus, are not available for other ligands. Accordingly, since the receptor or its active domains are covered no receptor-mediated functions can occur.

According to another embodiment the molecule modulating the PVR function binds to the PVR, preferably - but not exclusively - to an extracellular part of the PVR, in a way that the only one or more active domains of the receptor are bound by the molecule and, thus, blocked from interacting with other ligands. Accordingly, no naturally occurring receptor-mediated functions can be induced.

According to another embodiment the molecule modulating the PVR function binds to the PVR, preferably - but not exclusively - to an extracellular part of the PVR, in a way that the molecule binds close to an active domain and, thus, interferes with and/or disturbs the binding of other ligands. Accordingly, no or only modulated receptor-mediated functions can occur.

According to another embodiment the molecule modulating the PVR function binds to the PVR, preferably - but not exclusively - to an extracellular part of the PVR, in a way that the molecule by binding rearranges extracellular domains of the receptor and, thus, interferes with and/or disturbs the binding of other ligands. Accordingly, no or only modulated receptor-mediated functions can occur.

According to another embodiment the molecule modulating the PVR function binds to the PVR, preferably - but not exclusively - to an extracellular part of the PVR, in a way that the molecule by binding destroys one or more active domains or even the whole receptor and, thus, interferes with and/or disturbs the binding of other ligands. Accordingly, no or only modulated receptor-mediated functions can be induced.

According to another embodiment the molecule modulating the PVR function binds to the PVR, preferably - but not exclusively - to an extracellular part of the PVR, in a way that the molecule by binding activates and increases the receptor mediated function(s).

According to another embodiment the molecule modulating the PVR function binds to the PVR, preferably - but not exclusively - to an extracellular part of the PVR, in a way that the molecule by binding promotes further binding of molecules to the receptor or one or more active domains thereof. In this case the receptor-mediated functions are increased, accelerated and/or prolonged.

The molecules as described can be identified and isolated according to the method described hereinafter.

It was one of the achievements of the inventors to provide a method, which allows identifying and further isolating molecules according to the present invention. For this they started with an amplifiable ligand-displaying unit (ALDU) to identify tumor specific ligands by contacting the ALDU with a cancer cell of interest.

An "ALDU" is a collections of molecules with dual function: they can bind to a target cell via a ligand; and they carry physically associated with the ligand an information about its identity, which allows individual units to be identified, and amplified. A "ligand" as used herein is a molecule displayable by an amplifiable ligand-displaying unit (ALDU). A ligand is that part of an ALDU through which the ALDU can bind to a target. Preferably it is a small chemical compound, a polypeptide, antibody or antibody fragment as defined above, an RNA-oligonucleotide or a DNA-oligonucleotide, whereby the oligonucleotide comprises more than 20 base units but less than 10,000, preferably less than 1,000 base units. A ligand can bind to an intra- or extracellular region of an antigen. This binding may have specificity in the sense that the ligand binds to one antigen with high affinity but to a moderately related antigen with lower, for example 10- or 50- or up to 200-fold lower affinity. "Moderately related antigens" are antigens or receptors with up to 30 % amino acid identity in the extracellular regions.

Examples of ALDUs are oligonucleotides, particularly RNAs, useful for the process of selection-amplification (SELEX), phages of phage display libraries, viruses of viral display libraries, ribosomes of ribosome-display techniques, but also individualized beads carrying an identifiable molecule or ligand, particularly chemical entities, e.g. small molecules bound to beads, which are recognizable by inert chemical tags. Preferred are such ALDUs, which comprise a nucleic acid as the identifiable component. Such ALDUs can be either amplified *in vitro,* e.g., by nucleic-acid amplification methods like RT-PCR, PCR, LCR and the like, or they can be amplified *in vivo,* for example an individual phage can infect a bacterium and yield, after several cycles of infection, millions of virtually identical progeny. A library of ALDUs is a collection of similar, but in general non-identical ALDUs, for example phages of a phage library that display different scFvs in the context of an otherwise identical phage surface.

A ligand "derived from an ALDU" as mentioned herein is a ligand, which had been displayed by such an ALDU, which had been selected by binding to the surface of a target cell. As an example, if the ALDU is a phage of a phage library displaying scFvs the "ligand derived from an ALDU" in this case is a polypeptide, namely a scFv. As another example, in the case of ribosomal display the ligand "derived from an ALDU" is the polypeptide, which was presented by the complex comprising the ribosome, the RNA and the polypeptide.

The invention provides in a further embodiment a method of identifying a ligand binding specifically to the extracellular region of PVR, wherein said ligand is capable of affecting a biological function, like adhesiveness and invasiveness, of PVR, comprising the steps of
a) contacting a library of amplifiable ligand-displaying units (ALDU) with a cancer cell, e.g. a sarcoma cell;
b) separating said cancer cell and the ALDUs bound thereto from ALDUs not bound to said cancer cell;
c) amplifying the ALDUs bound to said cancer cell;
d) identifying an ALDU or a ligand derived from an ALDU after step c), which affects said biological activity of PVR by a functional screening assay;
e) identifying an ALDU or a ligand derived from an ALDU after step d) capable of binding to PVR
f) determining the chemical identity of the ligand.

The preferred - but not the only possible - order of the steps is a, b, c, d, e and optionally f.

The method advantageously combines a screening step based on binding to the surface of a cancer cell with a screening step based on a functional assay. Therefore a library of ALDUs is brought in contact with a cancer cell in such a way that those ALDUs of the library with specificity for a surface antigen of the cancer cell can bind to structures associated with the surface of e.g. the cancer cell. For example phages of a phage library displaying antibodies or antibody fragments can be allowed to bind to cultured cancer cells or, as another example, RNA of an RNA-oligonucleotides library can be allowed to bind to such cancer cells.

As used herein, the term "associated with the surface of a cell" means that a biomolecule is either part of the surface of a cell, e.g. a constituent of the surface, or is binding to another biomolecule which is a constituent of the surface of a cell. For example, a transmembrane polypeptide, like a transmembrane receptor of a eukaryotic cell can be seen as a constituent of the surface if at least one part of it transverses the lipid bilayer of the cell membrane and is in direct contact with the lipids of the cell membrane. An example for a polypeptide that is binding to another biomolecule, which is a constituent of the surface of a target, is, for example, the beta-2-microglobulin of the class 1 major histocompatibility complex (MHC). In this case the beta-2-microglobulin binds non-covalently to the alpha or beta chain of the MHC - a transmembrane polypeptide, which is also regarded as being a molecule associated with the surface.

As used herein "contacting" of two components means allowing the two components to physically associate with one another and bind to one another.

"Separating" as used herein means the physical separation of two or more components, for example a cell with an ALDU bound thereto can be separated from a free ALDU by centrifugation, wherein the cell with the ALDU bound thereto is pelleted and the free ALDU is still in the supernatant.

"Amplifying" as used herein is any process that increases the number of ALDUs or ligands derived from ALDUs by at least a factor of two, preferably by a factor of 10, 100, 1.000, 10.000, 100.000, 1.000.000, 10.000.000 or even a billion. For example a single phage can be amplified by infecting a bacterium and the infected bacterium then produces several mostly identical copies of the infecting phage, or a DNA-molecule can be amplified by the process of PCR to yield 10⁴ or more mostly identical daughter DNA-molecules.

"Identifying" as used herein means locating or recognizing an individual component, for example an ALDU, with special properties and isolating said individual component.

"Determining the chemical identity of a ligand" as used herein means determining the structural composition of a ligand. For example, if the ALDU library is a phage library displaying scFvs, then "determining the chemical identity of a ligand" would mean to determine the sequence of the scFv polypeptide, for example by sequencing the region encoding the scFv of the phage from which it was derived.

"A screening assay" as used herein is aimed at detecting an individual, e.g. an ALDU, with special properties among other similar individuals with slightly different properties. In order to qualify for a screening assay at least 3, 4, 5, 6, 7, 8, 9, 10, 20, 30, 40, 50 or even 100 individuals must be tested in a certain functional assay to find an individual/individuals with the desired function detected among them.

In the separating step (b.) according to the present invention, the cancer cell and the ALDUs bound thereto are separated from unbound ALDUs, in order to select for those ALDUs with specificity for the cancer cell. The separation can be achieved, e.g., by removing the solution, in which the contacting step has been performed from cultured cancer cells grown adhesively in a culture flask and together with the solution the unbound ALDUs; or, as another example, cancer cells with RNAs of a SELEX library bound thereto can be pelleted by centrifugation, while the unbound RNAs remain in the supernatant as part of the solution, in which the contacting step had been performed. Alternatively, separation can be achieved by centrifugation, density centrifugation, filtration, FACS sorting, immunoprecipitation or immobilization onto a solid support. For example an ALDU bound to a cancer cell can be separated from unbound ALDUs making use of the fact that the complex of an ALDU bound to a target has different biochemical properties compared to an unbound ALDU. Those changed biochemical properties can be size, specific binding, charge density, density or the like.

Filtration can separate the bound ALDUs from the unbound ALDUs, e.g. if the size of the filter is such that ALDUs bound to the cancer cell are retained by the pores of the filter, while the unbound ALDUs can pass through the pores of the filter. Filtration by size is therefore useful if the target is large, like the cancer cell, and the ALDU is small, e.g. a phage, a virus, a nucleic acid or a ribosome displaying a ligand.

Density centrifugation separates biomolecules according to their density. Density centrifugation can be applied if the density of an ALDU bound to a target is different from that of an unbound ALDU. For example, the density centrifugation can be applied if an ALDU is dense, e.g. a ribosome displaying a ligand, and the target is not as dense, like a sarcoma cell or vehicles derived from the membrane of a cancer cell.

FACS can separate the complex of ALDU bound to a cancer cell from unbound ALDUs based on fluorescence of the target. FACS can be performed by labeling the cancer cell with a fluorescent dye and then passing the labeled cancer cell in suspending medium through a narrow dropping nozzle so that each cancer cell is in a small, separate droplet. A laser-based detector system is often used to excite the fluorescent dye and droplets with e.g. fluorescence-positive cancer cells are given an electric charge. Charged and uncharged droplets can then be separated as they pass charge plates and can be collected in two different tubes. In this way small droplets containing ALDUs that are bound to a cancer cell are separated from bulk solution containing unbound ALDUs.

Immobilization of the cancer cell onto solid support can be performed in order to separate ALDUs bound to the immobilized cancer cell from unbound ALDUs. Immobilization of the cancer cell onto solid support is often performed by using a surface activated solid support or specific binding of the solid support surface with the cancer cell using e.g. biomolecules with specific binding properties towards the cancer cell. In order to separate ALDUs that are bound to the immobilized cancer cell from unbound ALDUs, the immobilized cancer cell is separated from the bulk solution containing unbound ALDUs.

Separation of ALDUs bound to the immobilized cancer cells from the bulk solution containing unbound ALDUs can be performed by removing the bulk solution containing the unbound ALDUs from the immobilized cancer cells. For example, if the cancer cell is immobilized onto e.g. the well of a plastic micro titer plate via covalent interactions then the bulk solution containing the unbound ALDUs can be separated by removing the solution and washing the wells with washing solution.

In the amplification step (c.) according to the method of the present invention the ALDUs bound to the target are amplified, in order to obtain high enough concentrations of the ALDUs so that they are useful for being applied in a functional screen. This amplifying step makes use of the ALDUs' intrinsic property to be amplifiable. For example phages displaying, e.g., an antibody or antibody fragment, which have been separated from the non-bound phages in step b), but are still bound to the cancer cell, can be amplified by first recovering the bound phages, for example by eluting the phages from the cancer cells and then using the recovered phage eluate to infect bacteria, e.g. *Escherichia coli.* The amplification of the phages in *Escherichia coli* then leads to a significant increase in total phage number, but in such a way, that the concentration of those individual phages, which were capable of binding to the cancer cells, is dramatically increased. According to a preferred embodiment the step of amplification is realized by either eluting the ALDU bound to a cancer cell, or by lysing a caner cell under conditions where the ALDU remains amplifiable, and subsequent amplification. ALDUs bound to a cancer cells can be eluted from the cancer cells first, e.g. by use of solutions with low pH and/or high salt, as demonstrated in the Examples, and subsequently amplified, e.g. such that the eluted phages are used to infect *Escherichia coli* in order to produce large numbers of progeny phages. However, the elution step from the target, while it may be convenient, is not necessary, as, e.g., in the case of phage libraries even the phages still bound to a cancer cell, are capable of infecting *Escherichia coli* and thus capable of producing large numbers of identical progeny, and thus are amplifiable even when still bound to the cancer cell. Accordingly, the phages need not necessarily be removed from the target cell, as a phage still bound to a target cell can infect added bacteria and thus be amplified.

As another example, the mRNA of a ribosomal-display library, which is associated with the surface of a cancer cell by its attachment to a ribosome displaying the polypeptide corresponding to the mRNA can be directly used for RT-PCR, without the need of removing the intact ALDU from the target before amplifying the target. Thus, a step in which the ALDU bound to a target is recovered from the target before amplifying the ALDU is an optional step.

Amplifying the ALDU can also consist in determining the chemical identity of the ligand of such ALDUs, which are bound to the cancer cell and then amplifying the ligands. That is to say, e.g., that in the case of a phage library, displaying an antibody or an antibody fragment, DNA encoding those ligands can be cloned from phages, which are bound to the cancer cell, e.g., by PCR, and the ligand, e.g., the antibody or antibody fragment, can then be produced recombinantly to yield sufficient amounts of the ligand to be useful for a functional screening assay. In such a way, not the ALDU itself is amplified, but the ligand displayed by the ALDU. This, however, is within the scope of the invention, as the aim of the amplification step is the generation of a ligand concentration high enough to be useful for a functional screening assay and this can be achieved by either amplifying the ligand or the ALDU displaying it.

In the identifying step (d.) according to the method of the present invention, an ALDU or a ligand derived from an ALDU, which had bound to a cancer cell is identified based on its effects on a biological function of PVR to be tested in a functional screening assay. For such a functional screening assay, the ALDUs or ligands derived from them are advantageously individualized, such that a signal or a pattern from the functional screening assay is relatable to an individual ALDU or an individual type of ligand. This can be done, e.g. by filling separate wells of a multi-well plate with individual ligands each and then performing an assay for a desired biological function of PVR in those individual wells.

In another example phages, which had bound to a cancer cell, and had been separated from unbound phages, can be individualized by infecting bacteria and plating the infected bacteria in, e.g. soft agar, so that individual plaques form, which represent clones of individual phages. Those individual plaques can then be tested for their effects in a biological screening assay for PVR function. In such an assay, an individual plaque with an effect in the functional screening assay for PVR function is identifiable. The ligand displayed by the phages of the plaque can then be identified, e.g. by sequencing the phage DNA of an isolated plaque, which represent a clone of a single phage, initially selected by its ability to bind to the cancer cell. Thus, in those cases where an ALDU itself is used in the functional screening assay, the identity of the ligand displayed by the ALDU can be determined, if desired.

In a preferred embodiment the ligand is capable to modulate and/or inhibit a biological function of PVR. For example, the biological function of PVR can be invasion, adhesion, or angiogenesis.

The term "invasiveness" as used herein is the ability of a cell to migrate through a layer of other cells or to migrate through the extracellular matrix. Invasiveness can be assessed by the Matrigel assay as described in the Examples. According to the present invention invasion is measured as cells that reach the lower surface of the filter during a certain incubation period. When more than 40% of cells within 6 to 12 hours reach the other side of the filter and form colonies in an invasion assay like the one described in the Examples, the naturally occurring cancer cell is defined as invasive. The control cells forming only 5% colonies in the same time frame are defined as non-invasive.

The term "adhesiveness" as used herein is the ability of a cell to reattach after they have been removed from the matrix on which it had been grown, re-suspended as a solution of single cells (not in direct contact with other cells of the solution), and replated on a matrix to which adhesion is possible. A cell is defined as adhesive if in an assay as described in the Examples, more than 40% of the cells adhere within a time of 30 to 120 minutes. In contrast, only 5% of the control cells adhere within the same time frame.

The term "angiogenesis" is the process where cells induce formation of new blood vessels in their proximity. Angiogenesis can accompany the growth of malignant tissue and can therefore be a property of malignant cells. That is to say that malignant cells can have the property of inducing angiogenesis.

In another embodiment the ALDU or the ligand derived from an ALDU and used for the functional screening assay as in step d) of the method according the invention identifying an unmodified ligand according to the invention.

In still another embodiment of the invention the ALDUs or the ligands derived from an ALDU which are used for the functional screening assay according to step d) of the method of identifying a ligand according to the invention are chemically modified to increase their biological activity or endow the ALDU or the ligand with biological activity. As an example, one way to increase the biological activity of an ALDU or a ligand or to endow the ALDU or the ligand with biological activity is to use the ALDU or the ligand in combination with Chromophore-Assisted Laser Inactivation (CALI) as described later on. CALI can also be used to further enhance the inhibitory effect of a ligand that has already an neutralizing and inhibitory effect by itself.

The chemical modification of the ALDU or the ligand derived from an ALDU can be the addition of chromophores. A chromophore is that part of a molecule that possesses high optical activity due to mobile electrons that interact with light. Some examples of chromophores are, e.g., fluorescein derivatives, rhodamine derivatives, coumarin derivatives, porphyrin derivatives, phthalocyanine derivatives, naphthalocyanines derivatives eosin derivatives, triphenylmethane derivatives or acridine derivatives. A list of chromophores useful for chemically modifying biomolecules is disclosed in The Sigma Aldrich Handbooks of Stains and Dyes, Ed., F.J. Green (1990) ISBN No. 0-941633-22-5.

Preferably, the biological function - such as adhesiveness and invasiveness of a cancer cell - to be inhibited by a ligand, e.g. an antibody or antibody fragment, is identified by the method of the present invention.

The term "inhibition" is understood to be at least a 20%, preferably a 30%, more preferably a 50%, even more preferable a 60% decrease in function, as defined by PVR function in an invasion assay as mentioned above, when compared to a negative control with the same experimental conditions, but without the molecule of the invention. A molecule is defined as not significantly affecting the function of the other three receptors if the decrease in function affected by the molecule of the invention is less than 20%, more preferably less than 15%, even more preferably less than 10%.

Additionally, the polypeptide, the antibody or antibody fragment according to the present invention is considered to inhibit the biological function of PVR if a reduction of the adhesiveness and of the invasiveness of naturally occurring cancer cells in an experiment by more than 20%, preferably more than 60%, can be detected.

Additionally, in the case of a molecule of the invention which inhibits gene expression of PVR, such a molecule decreases PVR expression by more than 50%, preferably by more than 80%, still more preferably by more than 90%, most preferably by more than 95% when measured in a quantitative western blot normalized to the level of beta tubulin, when present in an experiment at a concentration of 10 nM to 100 µM, preferably at around 1 µM, in which the amount of PVR is compared between two otherwise identical samples, wherein in one sample the molecule of the invention was allowed to inhibit PVR expression. In the same experiment the molecule of the invention does not decrease the amount of the beta tubulin present per cell by more than 20%, and said molecule does not decrease the relative level of the insulin receptor and the B-CAM cell surface glycoprotein by more than 20%.

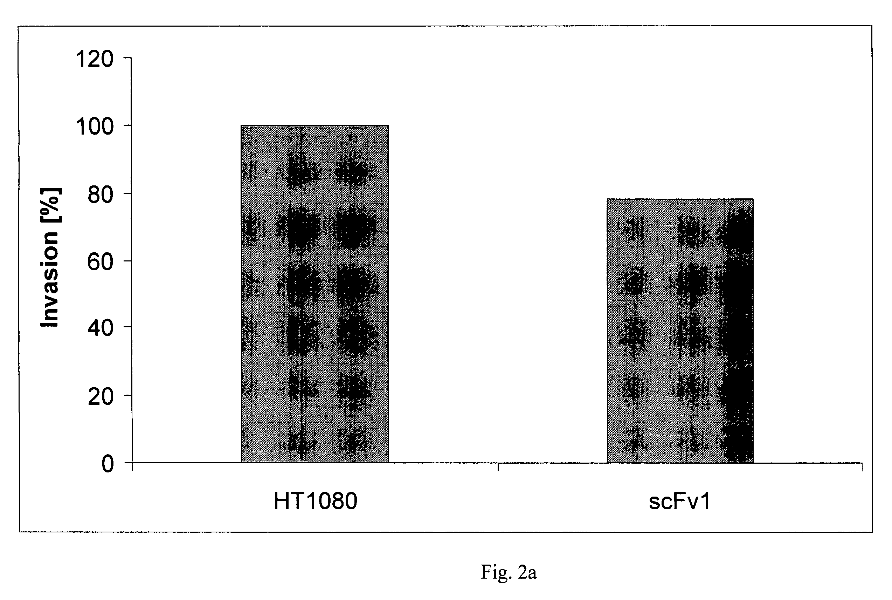

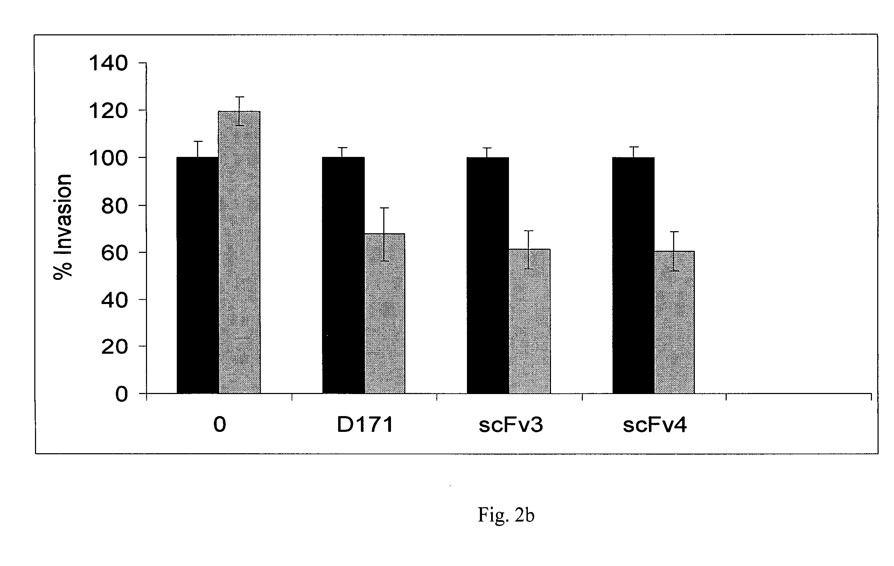

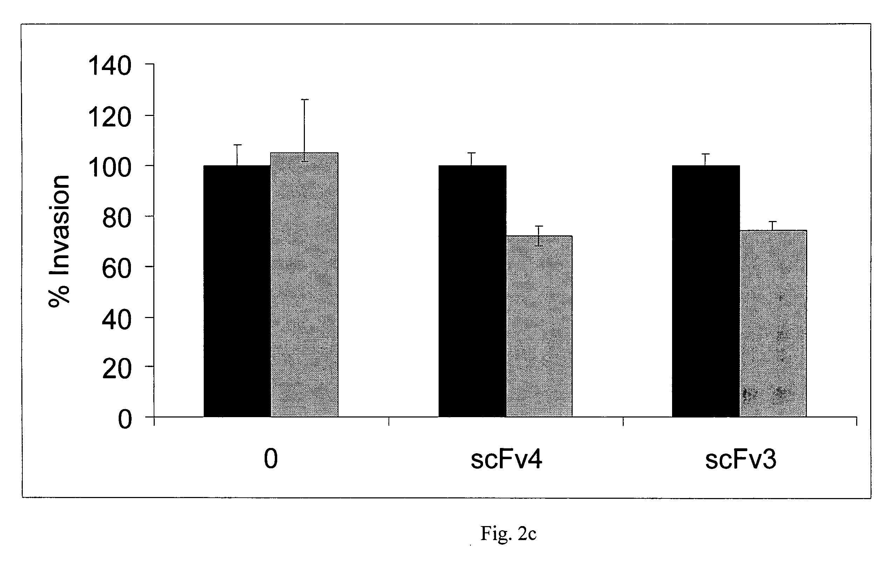

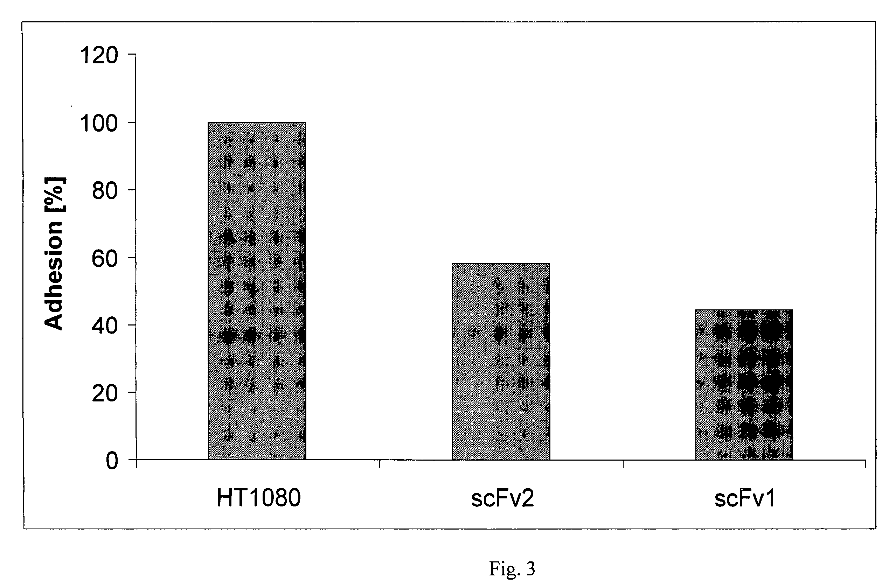

With the method, the inventors provided for the first time a possibility to identify and subsequently isolate molecules, which modulate PVR function(s). Furthermore, by attaching selected labels, which can be induced on command, e.g. a laser or a light beam, the inventors were able to modify the selected molecules in a way that the selected molecules were able to inhibit the PVR mediated adhesion behavior of a cell by about 41% to 55% (see, Figure 3). Furthermore, the PVR mediated invasion behavior of cells was inhibited by at least 25% compared with untreated cells (Figures 2a, 2b or 2c).

This makes also clear that the nature of the ligand displayed on the ALDU is not critical to identify suitable molecules, it is more the ability of the ALDU to be identifiable and amplifiable, which has influence on a successful screening for PVR modulating molecules according to the invention.

However, as explained before, amplification of the ALDU can be achieved by increasing the number of an individual ALDU, but also by increasing the number of a ligand displayed by an individual ALDU. Since this can in principle also be achieved by an individual bead displaying a small chemical in combinatorial chemical libraries (see Ohlmeyer et al. (1993) Proc. Natl. Acad. Sci USA 90, 10922-26; Liu et al. J. Am. Chem. Soc. (2002) 124, 7678-80; Liang et al Science (1996) 274, 1520-2) such bead-format libraries are also libraries of ALDUs. Small molecules can be synthesized on microsphere beads, which can be indexed with inert chemical tags. During each step of the synthetic scheme a tag molecule that encodes the step number and the chemical reagent used in that step is attached to the bead. This array of tags can then be used as a binary code to record the reaction history of each bead. The identified compound can then easily be synthesized in a bigger scale.

Protein scaffolds with the immunoglobulin fold are antibodies and antibody fragments and other polypeptides having the tertiary structure of the immunoglobulin superfamily and ALDUs displaying protein scaffolds with the immunoglobulin fold are disclosed in McCafferty et al. (1990) Nature 348, 552-4. ALDUs displaying random polypeptides are disclosed in Scott et al. (1990) Science, 249, 386-90. RNA-aptamers are structural mimics composed of ribonucleic acid and are disclosed in Tuerk and Gold ((1990) Science 249, 505-10), and Tuerk et al. ((1992) Proc. Natl. Acad. Sci. USA 89, 6988-92). DNA-aptamers are structural mimics composed of deoxyribonucleic acid and ALDUs displaying DNA-aptamers are disclosed in Bock et al ((1992) Nature 355, 564-6). An Affibody is a disulfide bond-independent three-helix bundle scaffold Z, derived from domain B of Staphylococcal protein A and ALDUs displaying Affibodies are described in Hansson et al ((1999) Immunotechnology 4, 237-52). A cysteine knot is a protein scaffold involving interlocking disulfide bonds as exemplified by the structure of conotoxins and nerve growth factor. A cysteine knot is described in McDonald et al. ((1991) Nature 354, 411-4) and ALDUs displaying cysteine knots are described in Smith et al. (J Mol Biol. (1998), 277, 317-32).

However, preferably the ALDU used according to the invention is a biological ALDU whereby the information about its identity is stored as a nucleic acid, e.g. RNA or DNA. In a preferred embodiment the ALDU is selected from the group consisting of viruses useful for viral display, phages useful for phage display, bacteria useful for bacterial display, ribosomes useful for ribosome display, yeasts useful for yeast display and oligonucleotides useful for selection and amplification. Viruses useful for viral display can be, e.g., plant viruses, viruses of fungi or animal viruses. Viruses that can be obtained from cell culture and that are amenable to genetic engineering techniques can display a foreign ligand on their surface. Viruses useful for viral display on retroviruses are disclosed in Russell et al., (1993) Nucleic Acids Res. 21, 1081-5. Viruses useful for display by plant viruses, e.g. the Cowpea mosaic virus are disclosed in Brendan et al., (1999) Microbiology 145, 211-20.

Phages useful for phage display can be all phages that can be obtained from culture and that are amenable to genetic engineering techniques and are capable of displaying a foreign ligand on their surface. Phage display has been disclosed in Smith et al. (1985) Science, 228, 1315-17, phage display of an antibody has been disclosed in WO 91/17271.

Bacteria useful for bacterial display are bacteria, which can be kept in culture, which are amenable to genetic engineering techniques and which are able to display a ligand on their surface. Examples for bacteria useful for bacterial display are disclosed in Dougherty et al., (1999) Protein Eng. 12,613-21; and Westerlund-Wickstrom B. (2000) Int. J. Meth. Microbiol. 290, 223-30.

Ribosomes useful for ribosomal display have been disclosed in Shaffizel et al., (1999) J. Immunol. Methods 231, 119-35; and Willson et al., (2001) Proc. Natl. Acad. Sci. USA 98, 3750-5.

Oligonucleotides useful for selection/amplification (Selex) have been disclosed in Tuerk and Gold (1990) Science 249, 505-10, and Tuerk et al., (1992) Proc. Natl. Acad. Sci. USA 89, 6988-92.

However, also lower eukaryotes which can be cultured and are amenable for genetic engineering techniques and, thus, able to display a foreign ligand on their surface are useful as ALDUs, e.g. genetically modified yeast, as disclosed in Boder and Wittrup (2000) Methods Enzymol. 328, 430-44.

In another preferred embodiment the ALDU library is selected from the group consisting of immune or naive libraries, in particular wherein said naive library is a synthetic, semi-synthetic or natural library. This relates particularly to the case wherein the ligand displayed on the ALDU is an antibody or an antibody fragment. In the case of immune libraries the library members, e.g. antibody fragment genes, can be obtained from lymphocytes, e.g. B lymphocytes of immunized animals.

In the case of naive libraries the library members have not gone through the process of an active immune response. A naive library can be a natural library wherein the library members e.g. antibody fragment genes, can be obtained from lymphocytes, e.g. B-lymphocytes, of non-immunized donors. An example of a natural library is disclosed in Griffiths et al. ((1994) EMBO J. 13, 3245-60).

Depending on the way of its production the naive library can also be, e.g., a semi-synthetic library, in which library members are obtained by use of germline V-genes as starting material. The regions coding for one or several V-gene CDRs, encoded e.g. by CDR1 and CDR2 can be replaced by randomized oligonucleotides, or randomized oligonucleotides can be added to one or several CDRs, either at the beginning, as an insert into the CDR, or at the end of the CDR, and regions coding for one or several of the non V-gene encoded CDRs e.g. CDR3 can be added to the V-gene as randomized oligonucleotides. Semi-synthetic libraries are disclosed in Williamson et al (1993) Proc Natl Acad Sci U S A. 90, 4141-5.

Antibody-based naive libraries can also be synthetic libraries, e.g. in which library members are obtained by use of non-germline V-genes as starting material. The regions coding for one or several V-gene CDRs encoded e.g. by CDR1 and CDR2 can be replaced by randomized oligonucleotides, or randomized oligonucleotides can be added to one or several CDRs, either at the beginning, as an insert into the CDR, or at the end of the CDR, and regions coding for one or several of the non V-gene encoded CDRs e.g. CDR3 can be added to the V-gene as randomized oligonucleotides (Griffiths et al (1994) EMBO J. 13, 3245-60; Knappik et al. J Mol Biol. 296, 57-86).

In another preferred embodiment of the invention, the ALDUs are derived from viruses and phages, in particular from Cowpea mosaic virus, retroviruses, lambda phage, T7 phage, T4 phage and filamentous phages, more particularly derived from Ff phages. An ALDU library derived from Cowpea mosaic virus is disclosed in Brendan et al. (1999) Microbiology 145, 211-20. An example of an ALDU library derived from a retrovirus is disclosed in Russell et al. (1993) Nucleic Acids Res. 21, 1081-5.

An example of an ALDU library derived from lambda phage is disclosed in Maruyama et al. (Proc. Natl. Acad. Sci. U S A (1994) 91, 8273-7). An example of an ALDU library derived from T7 phage is disclosed in Danner et al. (Proc. Natl. Acad. Sci. U S A (2001) 98, 12954-9). An example of an ALDU library derived from T4 phage is disclosed in Efimov et al. (Virus Genes (1995) 10, 173-7). Often filamentous phages have been used as the basis for ALDU libraries (Phage Display of Peptides and Proteins: a laboratory Manual/ edited by Brian K. Kay, Jill Winter, John McCafferty. Academic Press 1996). The Ff phages of *Escherichia coli* have been proven particularly useful as phages capable of displaying random and foreign ligands on their surface (Smith, Science. (1985) 228, 1315-7). In those cases the ligand, e.g. a random polypeptide, is often displayed as a fusion protein with a phage protein, wherein the phage protein is derived from a phage surface protein, e.g. selected from the group consisting of pIII, pVIII and pVI. Phage display based on fusion proteins with pIII are disclosed in Smith (Science. (1985) 228, 1315-7). Phage display based on fusion proteins with pVIII are disclosed in Gram et al. (Proc. Natl. Acad. Sci. U S A (1992) 89, 3576-80). Phage display based on fusion proteins with pVI are disclosed in Jespers et al. (Biotechnology (NY). (1995) 378-82).

In another preferred embodiment step d) of the method of identifying a ligand of the invention comprises detecting an ALDU or a ligand derived from an ALDU, which affects the biological function of PVR by a functional screening assay, wherein the functional screening assay applied for detecting the ALDU or a ligand derived from an ALDU is selected from the group consisting of an angiogenesis assay, an invasion assay and/or an adhesion assay, as described thereafter.

The method of the invention, the method of identifying a ligand of the invention comprises as the identification step d) a spatial separation of the different ALDUs of a pool of ALDUs and then the screening of the spatially separated ALDUs for their effect on a biological function of PVR in such a way that the result obtained can be assigned to an individual ALDU. Spacial separation of the different ALDUs of a pool of ALDUs can be done, e.g., by filling separate wells of a multi-well plate with individual ligands each and then use those multi-well plates in a functional screening assay. If, e.g., in one well the biological function of PVR is inhibited, then a ligand inhibiting said biological function of PVR is identified, as the experimentator knows the identity of each ligand he put into each individual well.

In another preferred embodiment the method of identifying a ligand according to the invention can comprise the step of determining the identity of the ligand, wherein this determination step is achieved by sequencing the DNA of an identified ALDU, taking a PCR fingerprint of the nucleic acid of an identified ALDU or also, in the case of a ligand derived from an ALDU, by mass spectrometry of such a ligand. PCR fingerprinting of, e.g., phages is disclosed in Marks et al. (J. Mol. Biol. (1991) 222, 581-97). Mass spectrometrical analysis of a ligand, e.g. a polypeptide, is disclosed in Shevchenko et al. ((1996) Anal. Chem. 68, 850-58) or Spengler et al ((1992) Rapid. Commun. Mass. Spectrom. 6, 105-8.)

The method of identifying a ligand according to the invention may further comprise at least one additional step of screening ALDUs, e.g. wherein the screening is based on biochemical properties of the ALDUs. Such an additional screening step may comprise a method selected from the group consisting of flow cytometry, ELISA, immunoprecipitation, binding assays, immunohistochemical experiments, affinity studies, immunoblots and protein arrays. Biochemical properties can be determined by the size, the shape, the density, the charge density, the hydrophobicity, or the binding specificity of an ALDU or the ligand derived from an ALDU. The biochemical properties of the ALDU or the ligand derived from an ALDU form the basis of the applicability of the above-mentioned methods for screening the ALDUs. Flow cytometry is usually performed by labeling cells with a fluorescent dye and then passing those labeled cells in suspending medium through a narrow dropping nozzle so that each cell is in a small, separate droplet. A laser-based detector system is often used to excite the fluorescent dye and droplets with e.g. fluorescence-positive cells are registered.

The term "immunoprecipitation" describes a procedure by which a ligand, e.g. an antibody or antibody fragment, that reacts specifically with an antigen, e.g. an antigen associated with the surface of a eukaryotic cell, is used to remove said antigen from a solution, e.g. a cell lysate. The immunoprecipitate consisting of the ligand bound to the antigen can then be examined by utilizing biochemical methods like, e.g. 2D gel electrophoresis. In particular the identity of the antigen can then be determined by use of, e.g., mass spectrometry and it can be determined whether this ligand really binds to PVR.

In another preferred embodiment of the invention, the method of identifying a ligand of the invention can further comprise a substractive selection step. A substractive selection step is a step, which removes ALDUs with an undesired property. For example a substractive selection step can be affected by removing the ALDUs capable of binding to a control cell, if the property of binding to a control cell is undesired. By way of example, if one wants to select for ALDUs specific for cancer cells one could first select those ALDUs which bind to cancer cells, elute the bound ALDUs, e.g. phages, and then remove those ALDUs, which are capable of binding to non-cancer-cells, by for example contacting the pool of eluted ALDUs with non-cancer-cells and removing those ALDUs bound to the non-cancer-cells. The ALDUs remaining in the supernatant are then ALDUs specific for cancer cells and can then be used in functional screening assays according to the method of identifying a ligand of the invention.

In step e) the pool of ALDUs, e.g. phages, is enriched in ALDUs binding to PVR. Those ALDUs binding to PVR can finally be identified in step f) by numerous methods known in the art. ALDUs, e.g. phages, can be separated to form individual clones and the clones of the ALDUs, e.g. phages, can be probed with labeled PVR protein, or a labeled part of the PVR protein, e.g. an at least seven amino acid long peptide of the extracellular region of PVR. Clones binding to such a probe are identified as PVR binders. ALDUs, e.g. phages, can also be affinity purified on purified PVR protein or on recombinant PVR

Alternatively, the open reading frame encoding the ligand of the ALDU, e.g. an antibody or antibody fragment, can be recloned from the whole enriched pool into an expression vector, the ligand, e.g. an antibody or the antibody fragment can then be expressed in clones of another host cell, and the clone of the host cell carrying the expression vector comprising a nucleic acid encoding for the ligand, e.g. an antibody or the antibody fragment binding specifically to PVR can be identified, e.g. by the method described above for the identification of relevant phage clones, by the method of the Examples, or by affinity purification on recombinant PVR.

A particular advantage of this method is that a ligand, e.g. an antibody or an antibody fragment specific for the accessible part of the extracellular region of PVR is obtained, since the initial selection step is performed on intact cells, which present the accessible part of the extracellular region of PVR for binding of the ligand.

In a preferred embodiment of the invention, the above method comprises instead of steps e) and f) the further steps of:
e) contacting ALDUs, e.g. isolated phages with recombinant PVR;
f) washing said PVR with a buffered detergent and/or high salt solution; and
g) eluting ALDUs, e.g. phages bound to PVR; and
h) determining the identity of the ligand, e.g. an antibody or antibody fragment represented by said eluted ALDU, e.g. a phage.

The "detergent" used can be a detergent solution, preferably buffered, and can be Tween in a concentration of 0.001- 0.5%, particularly 0.01-0.1 %. "High salt" as used herein means a high salt solution, preferably buffered, and has an ionic strength of 10 mM-1M, particularly 20-500 mM, more particularly 50-350 mM, even more preferably 80-250 mM. Typical useful anions are, for example, chloride, citrate, phosphate, hydrogen phosphate or borate. Typical useful cations are, for example, sodium, potassium, lithium, calcium or magnesium.

The buffered solution in the above paragraph typically has a pH of 7-8. For example, DMEM or PBS, particularly with 1-20%, more particularly 5-15%, even more preferably about 10% FCS, can be used as buffers.

Isolation of cells with phages bound to them is effected by gentle centrifugation at g values from 200 to 300 for 3 to 20 minutes, particularly 5 to 10 minutes. Elution of bound phages, both to cells and to immobilized PVR, is effected by a wash with 2-100 mM, particularly 4-50 mM, more particularly 5-20 mM, even more preferably around 10 mM Glycine at a pH of from 0 to 2.5, particularly from 1 to 2.5, more particularly from 1.5 to 2.5.

According to an embodiment of the present invention molecules identified and/or isolated due to their specific binding capacity to PVR and due to their ability to modulate PVR functions are selected from groups comprising small chemical compounds, oligonucleotides, peptides, oligopeptides, polypeptides, proteins, antibodies, antibody fragments, anti-idiotypic antibodies and/or bioconjugates.

A "small chemical compound" as used in this invention is a molecule with a molecular weight between 50 Da and 10000 Da, preferably between 100 Da and 4000 Da, more preferably between 150 Da and 2000 Da, or a physiologically acceptable salt thereof. Additionally, in the case of a small chemical compound of the invention, said compound is considered to modulate or inhibit the biological function of PVR if it reduces the adhesiveness of naturally invasive cancer cells by more than 30%, preferably by more than 60% and thereby not affecting cell morphology, cell cycle progression (determined by analyzing the DNA content of a cell population by propidium iodide staining and FACS analysis), and not increasing the percentage of the cells of the culture that show signs of apoptosis (determined by measuring the percentage of cells showing DNA fragmentation, e.g. by a so called tunnel-assay).

A "polypeptide" as used herein is a molecule comprising more than 10, preferably more than 20, most preferably more than 30, and less than 10000, more preferably less than 2500, most preferably less than 1000 amino acids. Also polypeptides, which contain a low percentage of modified or non-natural amino acids, are encompassed.

For amino acid sequence above 10000 residues the term "protein" is used hereinafter, for amino acid sequence with less than 10 residues the term "peptide" is used. Also peptides or proteins, which contain a low percentage of modified or non-natural amino acids, are encompassed.

The terms "antibody" and "immunoglobulin", as used herein refer to any immunological binding agent, including polyclonal and monoclonal antibodies. Normal antibodies have a common core structure of two identical light chains and two identical heavy chains. One light chain is attached to each heavy chain and the two heavy chains are attached to each other. Both the light and the heavy chains contain series of repeating, homologous units each about 110 amino acid residues in length, which fold independently in the so-called immunoglobulin (Ig) domain. To provide an unlimited and high specificity of antibodies against antigens and the correlating diversity of structure the N-terminal end of the light and heavy chains encompasses a so-called variable (V) region to distinguish from the more conserved constant (C) region of the remainder of each chain. Each V comprises three hypervariable regions, which are called also "complementarity-determining regions" (CDR) since these sequences form a surface complementary to the three-dimensional surface of the bound antigen. The CDRs of an antibody are the parts of these molecules that determine their specificity and make contact with specific ligands. The CDRs are the most variable parts of the molecule and contribute to the diversity of these molecules. They are structurally defined in a human IgG as amino acids 24 to 41 (CDR-L1), 50 to 57 (CDR-L2) and 90 to 101 (CDR-L3) of the light chain and amino acids 26 to 38 (CDR-H1), 51 to 70 (CDR-L2) and 100 to 125 (CDR-H3) of the heavy chain (see Kabat et al. (1987) 4th edn US Department of Health and Human Services, Public Health Service, NIH, Bethesda). The CDR regions of an antibody fragment can easily be determined by somebody skilled in the art by aligning the antibody fragment with said human IgG, e.g. using a program of the NCBI that allows to "Blast", and thereby align, two sequences with one another, and identifying the amino acids of the antibody fragment corresponding to the CDRs of a human IgG.

Antibodies according to the present invention may be also selected from modified immunoglobulins, for example chemically or recombinantly produced antibodies, CDR grafted antibodies or humanized antibodies, site-directed-mutagenized antibodies.

In a further embodiment the molecule of the invention is an antibody fragment, in particular a single chain antibody fragment (scFv), dsFv, Fab', Fab, F(ab')2, Fv, single domain antibody and/or diabody.

The term "antibody fragment" is used to refer to any fragment of an antibody-like molecule that has an antigen binding region, and this term includes antibody fragments such as scFv, dsFv, Fab', Fab, F(ab')₂, Fv, single domain antibodies (DABs), diabodies, and the like. The techniques for preparing and using various antibody-based constructs and fragments are well known in the art (see Kabat et al. (1991) J. Immunol. 147, 1709-19).

"Single chain antibody fragments" (scFv) comprise the variable domain of the heavy chain (VH) and the variable domain of the light chain (VL) of an antibody, wherein these domains are present in a single polypeptide chain. Generally, the scFv polypeptide further comprises a polypeptide linker between the VH and VL domains that enables the scFv to form the desired structure for antigen binding.

A "Fv" fragment is the smallest antibody fragment that retains an intact antigen binding site. A "dsFv" is a disulfide stabilized Fv. A "F(ab')₂" fragment, is a bivalent fragment comprising two Fab fragments linked by a disulfide bridge at the hinge region. A "Fab" fragment is an antigen-binding fragment, containing complete light chains paired with the VH and CH1 domains of the heavy chain. A "Fab"' fragment is a reduced F(ab')₂ fragment.

A "single domain antibody" (DAB) is an antibody with only one (instead of two) protein chain derived from only one of the domains of the antibody structure. DABs exploit the finding that, for some antibodies, half of the antibody molecule binds to its target antigen almost as well as the whole molecule (Davies et al. (1996) Protein Eng. 9: 531-537.

"Diabodies" are bivalent or bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (Holliger et al. (1993) Proc. Natl. Acad. Sci. USA, 90, 6444-6448).

In another embodiment the molecule of the invention is a polyclonal or monoclonal antibody, in another embodiment the antibody is a human or humanized antibody. In still another embodiment the antibody may include as light chain one or two scFv antibody fragment according to the invention. In still another embodiment the antibody is a anti-human PVR binding antibody, which may be selected from modified immunoglobulins, for example chemically or recombinantly produced antibodies or humanized antibodies, site directed mutagenized antibodies, which retain substantially the same affinity for PVR.

In another embodiment the polypeptide of the invention is a human antibody selected from the group consisting of IgA, IgD, IgE, IgG, and IgM, in particular IgG and IgM, more particularly IgG1, IgG2a, IgG2b, IgG3, IgG4.

By using a suitable molecule of the invention, particularly the antibody fragment or antibody, it is further possible to produce anti-idiotypic antibodies according to the invention. Such anti-idiotypic antibodies can be produced using well-known hybridoma techniques (Kohler et al. (1975) Nature, 256:495). An anti-idiotypic antibody is an antibody, which recognizes unique determinants present on another antibody. These determinants are located in the hypervariable region of the antibody. It is this region, which binds to a given epitope and, thus, is responsible for the specificity of the antibody. An anti-idiotypic antibody can be prepared by immunizing an animal with the polypeptide, particularly the antibody fragment or antibody, of interest. The immunized animal will recognize and respond to the idiotypic determinants of the immunizing antibody and produce an antibody to these idiotypic determinants. By using anti-idiotypic antibodies, it is possible to identify other hybridomas expressing monoclonal antibodies having the same epitopic specificity. The anti-idiotypic antibodies according to the present invention can be used to screen antibodies and verify whether the antibody has the same binding specificity and functionality as a molecule of the invention.

It is also possible to use the anti-idiotype technology to produce monoclonal antibodies, which mimic an epitope. For example, an anti-idiotypic monoclonal antibody made to a first monoclonal antibody will have a binding domain in the hypervariable region, which is the "image" of the epitope bound by the first antibody. Thus, the anti-idiotypic monoclonal antibody can be used for immunization, since the anti-idiotype monoclonal antibody binding domain effectively acts as an antigen.

In another embodiment, the molecule of the invention can be covalently or non-covalently conjugated and/or coupled to or with, respectively, another protein, a solid matrix (e.g. like a bead), with itself to form multimers, a cytotoxic agent further enhancing the toxicity to targeted cells, a cytostatic agent, a prodrug, or an effector molecule, which is able to modify the cell expressing PVR or to recruit immune cells. All these conjugates are "bioconjugates" according to the invention.

The term "bioconjugate" according to the present invention comprises the molecule of the invention, particularly the antibody fragment or antibody of the invention, together with one or more cytotoxic moieties, which are made using a variety of bifunctional protein coupling agents. Some examples of such reagents are N-succinimidyl 3-(2-pyridyldithio)-propionate (SPDP), bifunctional derivatives of imidoesters such a dimethyl adipimidate HCl, active esters such as disuccinimidyl suberate, aldehydes such as glutaraldehyde, bisazido compounds such as his (R-azidobenzoyl) hexanediamine, bisdiazonium derivatives such as bis-(R-diazoniumbenzoyl) ethylenediamine, diisocyanates such as tolylene 2,6-diisocyanate, and bis-activated fluorine compounds such as 1,5-difluoro-2,4-dinitrobenzene. Methods useful for the production of bioconjugates are know by someone skilled in the art and described in detail in March's Advanced Organic Chemistry: Reactions, Mechanisms and Structure, 5th Edition, Wiley-Interscience; or Bioconjugate Techniques, Ed. Greg Hermanson, Academic Press.

A list of preferred cytotoxic agents to link to a bioconjugate of the present invention includes, but is not limited to, daunorubicin, taxol, adriamycin, methotrexate, 5 FU, vinblastin, actinomycin D, etoposide, cisplatin, doxorubicin, genistein, andribosome inhibitors (e.g. trichosantin), or various bacterialtoxins (e.g. Pseudomonas exotoxin; Staphylococcus aureus protein A).

In one embodiment such molecules are molecules or oligonucleotides, which modulate the PVR function by inhibiting gene expression of PVR, for example antisense oligonucleotides or interfering double stranded RNAs (siRNAs) or vectors that lead to the cellular production of such siRNAs. Additionally, such molecules bind specifically to already expressed PVR and inhibit PVR function related to adhesiveness, invasiveness and/or metastatic potential of cells.

Antisense oligonucleotides are well known in the art as means to reduce gene expression of a specific gene (see, for example, Probst J.C. (2000) Methods 22(3): 271-281; Heasman J. (2002) Dev. Biol. 243(2): 209-214; Castanotto et al. (2000) Methods Enzymol. 313: 401-420 and Jen and Gewirtz (2000) Stem Cells. 18(5): 307-319). Recently it has been found that short, double stranded RNAs potently and specifically inhibit expression of an mRNA with a complementary exonic sequence. This phenomenon has been termed RNA interference (RNAi). These double stranded RNAs have been termed siRNAs, for small interfering RNAs, have a paired region of at least 18, preferably at least 19 nucleotides (this paired, double stranded region targets them against an mRNA with a complementary exonic sequence), and have a length of 20 nucleotides to 25 nucleotides, preferably of 21 nucleotides to 23 nucleotides (Elbashir et al. (2001) Nature 411, 428-429). These siRNAs can be synthesized chemically by methods well known in the art and are commercially available (see e.g. suppliers given in Elbashir et al. above and also in Elbashir et al., (2002) Methods 26, 199-213) or by T7 transcription off a suitable DNA template (see Yu et al. (2002) PNAS 99, 6047-6052). They can be delivered to a wide range of cell types, in which inhibition of gene expression of a certain gene is desired, by, e.g., synthesizing the two RNA strands, annealing them and transfecting them (see Elbashir et al. and Yu et al., above). Detailed protocols for the application of siRNAs are described in Elbashir et al., (2002) Methods 26, 199-213.

Another way of effecting RNA interference is to transfect a plasmid that leads to the cellular production of siRNAs or siRNA like hairpin RNAs, which are also functional in RNAi. This can be done by plasmids which carry both, the sense and the antisense strand of the siRNA under the control of a mammalian, preferrably human or mouse, U6 promotor, which leads to the transcription of both strands in a transfected cell, some of which anneal to form functional double stranded siRNAs within the transfected cell (Miyagishi and Taira (2002) Nature Biotech. 19, 497-500). Alternatively, the RNA species transcribed from the U6 promotor can be siRNA-like hairpin RNAs which consist of a 19-base pair siRNA stem with the two strands joined by a structured loop and a U₁₋₄ 3' overhang at the end of the antisense strand (Paul et al. (2002) Nature Biotech. 19, 505-508). Alternatively the RNA species transcribed from the U6 promotor can be short hairpin RNAs, which are transcribed as small temporal RNAs, short hairpin precursors of about 70 nucleotides, and processed into active siRNAs within the cell in which they are transcribed (Paddison et al. (2002) Genes and Development 16, 948-958). All these methods based on transfection of plasmids have in common that they lead to the cellular production of siRNAs, which leads to the inhibition of the gene with an exonic region complementary to the at least 18 nucleotide long base paired region of the siRNA. It is clear that also future ways to affect the cellular presence of siRNAs will lead to the desired effect of inhibiting PVR function and are within the scope of the invention.

In another embodiment the molecule of the invention is selected from the group consisting of protein scaffolds with a tertiary structure, random polypeptides, small molecules, RNA-aptamers and DNA-aptamers. Protein scaffolds with tertiary structure are molecules obtainable by using a gene encoding a polypeptide with a defined tertiary structure, wherein one or more regions of the gene encoding the polypeptide can be replaced by randomized oligonucleotides or randomized oligonucleotides can be inserted into the gene encoding the polypeptide without changing the tertiary structure of the encoded polypeptide. Examples for these molecules are e.g. Anticalin, Affibody, cystein knot or protein scaffolds with an immnoglobulin fold. Anticalin is described in Beste et al (1999) Proc Natl Acad Sci USA. 96, 1898-903.

In another embodiment the molecule of the invention is labeled with a detectable label. The terms "label" or "labeled" refers to a detectable marker or the incorporation of such, respectively, e.g., by incorporation of a fluorophore-, chromophore- or radio-labeled amino acid or attachment of a fluorophore-, chromophore- or radiolabel to a polypeptide or attachment of moieties that can be detected by a labeled second molecule containing a fluorescent marker or enzymatic activity that can be detected by an optical or a colorimetric method. An example for such a two-step detection system is the well-known biotin-avidin system. Various methods of labeling polypeptides and glycoproteins are known in the art and may be used (Lobl et al. (1988) Anal. Biochem., 170, 502-511). Particularly, examples for detectable labels according to the present invention are radioisotopes, chromophores, fluorophores, enzymes or radioisotopes.

According to another embodiment the molecule of the invention is labeled and/or chemically modified with an inducible label to increase its biological activity. The induction of the chemical label can be activated by temperature rise, laser light induction or any other high energetic wave. According to one further embodiment of the present invention the molecules are chemically labeled with e.g. one or more chromphoric agent as described above to increase the biological activity of a ligand in combination with Chromophore-Assisted Laser Inactivation (CALI).

The principle of CALI is based on the local initiation of a photochemical reaction that leads to the generation of short-lived reactive species, which in turn selectively modify the target molecule and cause its functional inactivation. Highly specific but non-inhibitory ligands (e.g. antibodies, antibody fragments, small molecules) are labeled with a suitable chromophore (e.g. malachite green, fluorescein, methylene blue, eosin). After complex formation between the target molecule (e.g. proteins) and the ligand, the complex is irradiated with light (laser or visible light) of an appropriate wavelength to excite the chromophore. The excitation triggers a photochemical reaction that initiates the generation of short-lived reactive species (e.g. hydroxyl radicals or highly reactive oxygen species). These reactive species modify the protein within a small radius around their site of generation. The distance that a reactive species can travel is very short due to its short lifetime. Therefore, the modifications of amino acid residues within the protein occur in close proximity to the binding site of the ligand. The damaging effect is restricted to a radius of 15-40 Å, which is well below the average distance of two proteins within a cell, which is at about 80 Å, (assuming an average cytosolic protein concentration of 300 mg/ml and an average protein size of 50 kDa) ensuring a high spatial resolution of the process. In cases where the binding site of the ligand is close or within an important functional domain of the protein, the induced modifications lead to permanent inactivation of the protein. The functional inactivation of the protein is measured in an appropriate readout assay and evaluated in the context of disease relevant physiological functions like cell invasion, cell adhesion cell signaling or apoptosis. Therefore, these ligands can be used to modulate the action of inhibitory ligands.

In one embodiment the modified polypeptide or the bioconjugate of the invention binding to human PVR, reduces the adhesiveness and/or invasiveness of human cancer cells by 20-60%, or preferably by 30-55%, 40-50% or even at least 60%, when tested in an adhesion assay or an invasion assay (Examples).

As a further example, a modified antibody against a given protein often can selectively inhibit the function of that particular protein after CALI, even if this antibody did not show an inhibiting function without CALI. In cases where the binding site of the ligand is close or within an important functional domain of the protein, these induced modifications lead to permanent inactivation of the protein. The functional inactivation of the protein is measured in an appropriate readout assay and evaluated in the context of disease relevant physiological functions like cell invasion, cell adhesion, cell signaling or apoptosis. It was shown by the inventors that CALI is able to convert specific but non-inhibitory ligands into blocking reagents (Examples).

According to a further embodiment of the invention cells expressing CD155, PVR or any derivative thereof are preferably proliferating cells, which are part of or involved in a proliferative disorder or disease. For example such cells are cancer cells, cells derived from a primary or metastatic tumor or from micro metastases. Furthermore, proliferative cells according to the invention are cells having a metastatic potential, e.g. cells having an increased readiness to traffick to and invade into distant tissue. In still another embodiment according to the present invention the cells expressing PVR, CD 155 or any of their derivatives originate from or derive from naturally occurring tumors or cancers.

A "cell originating from or deriving from a naturally occurring cancer " as used herein is a cell that has not been transfected, transduced or otherwise genetically engineered in the laboratory. Such a cell does not comprise any artificial DNA sequences, e.g. of vectors or DNA sequences being found only in other species, but not usually in the species from which the naturally occurring cancer cell was derived. However, a naturally occurring cancer cell may comprise sequences that are not usually found in the species from wherefrom it was derived, if those sequences have arisen due to the processes of mutation, viral infection and/or selection that took place within the individual from which the naturally occurring cancer cell was derived, and/or obtained during continued culture of the naturally occurring cancer cell.

"Metastatic tumors" as used herein includes both tumors at the primary site capable of metastasizing and metastasized tumors at a secondary site. Such metastatic tumors can be of any organ or tissue origin, like brain, central nervous system, lungs, stomach, lower intestine, liver, kidneys or the pancreas, etc., in particular tissue of mesodermal origin such as bone, spleen, thyroid, endometrial, ovarian, or lymphoid tissue. "Metastatic potential" as used herein is the ability of a tumor cell to form a new tumor at a site distant from the primary tumor of which the tumor cell was derived (a metastase). Metastatic potential can be measured by injecting, e.g. 1x10⁶, cells into the lateral tail vein of athymic nude mice and determining the number of tumor nodules in the lung, e.g. 2 months post injection, e.g. as described in the section "Tumor cell injections" on page 2346 of Huang et al (1996) Oncogene 13, 2339-2347, or the sections "Animals and production of tumors" and "Histochemical analysis for calcified matrix" on page 1882 of Radinsky et al. (1994) Oncogene 9: 1877-1883. A cell line to produce more than 3, preferably more than 8, more preferably more than 20 tumor nodules in the lung in this assay is considered metastatic.

A "micro-metastase" is an accumulation of tumor cells with a size smaller than 2 mm, which can usually only be detected by histological methods.

According to a further embodiment the proliferating cells according to the present invention are derived from selected cancer cell types such as listed below.

Selected "cancer cell-types" as preferably screened according to the methods of the invention as well as preferably treated according to the invention are selected from the group comprising astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, melanoma, cholangiocarcinoma, synovioma, mesothelioma, Ewing's tumor, rhabdomyosarcoma, coloncarcinoma, basal cell carcinoma, sweat gland carcinoma, papillary carcinoma, sebaceous gland carcinoma, papillary adenocarcinoma, cystadenocarcinoma, medullary carcinoma, bronchogenic carcinoma, renal cell carcinoma, bileduct carcinoma, choriocarcinoma, seminoma, embryonal carcinoma, Wilms' tumor, testicular tumor, prostate, breast, medulloblastoma, craniopharyngiorna, ependymoma, pinealoma, hemangioblastoma, acoustic neurorna, oligodendroglioma, meningioma, neuroblastorna, retinoblastoma, leukemia, multiplemyelorna, Waldenstrom's macroglobulinemia, and heavy and light chain disease, and adenocarcinomas of the uterine cervix, uterine and ovarian epithelial carcinomas, transitional cell carcinoma of the bladder, B and T cell lymphomas (nodular and diffuse) plasmacytoma, acute and chronic leukemias, soft tissue sarcomas, leiomyosarcomas or any other tumor cell with high PVR expression.

According to a further embodiment and as exemplatory result of one experimental set up, as described herein and in the examples, a scFv having the amino acid sequence SEQ ID No.: 3, SEQ ID No.: 4, SEQ ID No.: 69 or SEQ ID No.: 70 or an CDR having the amino acid sequence LWLRRD (SEQ ID No.: 1) or WTGDFDY (SEQ ID No.: 2) and specifically binding to PVR as well as modulating its function was isolated.

In another embodiment of the invention the molecule, polypeptide and/or the bioconjugate of the invention are used for identifying additional molecules that specifically bind human PVR in screening assays. These methods entail contacting a reference anti-PVR molecule or antibody fragment with a target species comprising the PVR domain in the presence of a putative competitor test-binding agent. This step of contacting is conducted under conditions suitable for complex formation between the reference antibody fragment and the target species in the absence of the test-binding agent. Complex formation between the reference antibody fragment and the target species in the presence of the test-binding agent is detected as an indicator of specific binding activity of the test-binding agent to PVR. This screening method is useful for high throughput screening of, e.g., other antibody libraries or antibody fragment libraries, antisense oligonucleotide libraries or peptide and small molecule libraries to identify and characterize additional molecules binding specifically to PVR. Competition is determined by an assay in which the antibody fragment, or other binding agent under test substantially inhibits specific binding of the reference antibody fragment to the target species containing the PVR domain. This can be determined for example by measuring binding of the reference antibody fragment to a target species comprising PVR domain in the presence and absence of a putative competitor, i.e. a molecule binding specifically to PVR under conditions suitable for complex formation.

Numerous types of competitive binding assays are known and routinely practicable within the invention, as described for example in US 4,376,110. Typically, such assays involve the use of a target species containing the PVR domain (e.g., purified PVR or a cell line expressing the PVR antigen), an unlabeled molecule binding specifically to PVR, and a labeled reference antibody fragment or other binding agent. Competitive inhibition is measured by determining the amount of label bound to the target species in the presence of the molecule binding specifically to PVR. Usually the molecule binding specifically to PVR is present in excess. Molecules binding specifically to PVR identified by these competition assays ("competitive binding agents") include antibodies, antibody fragments, peptides, antisense oligonucleotides, small molecules and other binding agents that bind to an epitope or binding site bound by the reference antibody fragment, as well as a molecule binding specifically to PVR that bind to an epitope or binding site sufficiently proximal to an epitope bound by the reference antibody fragment. Preferably, competitive binding agents of the invention will, when present in excess, inhibit specific binding of a reference antibody fragment to a selected target species by at least 10%, preferably by at least 25%, more preferably by at least 50%, and even more preferably by at least 75%-90% or greater. In addition to a polypeptide of the invention, particularly a modified antibody fragment or a modified antibody of the invention, natural or artificial ligands, peptides, anti-sense, or other small molecules capable of specifically targeting human PVR may be employed.

The present invention further relates to a method to amplify the molecule of the invention, including an antibody fragment, more particularly a scFv, dsFv, Fv, single domain antibody or diabody of the invention by recombinant techniques. These techniques are well known in the art (Skerra et al. (1993), Curr. Opin. Immunol. 5, 256-62; Chadd et al. (2001), Curr. Opin. Biotechnol. 12, 188-94).

For example, nucleic acid sequences encoding a polypeptide of the invention, particularly an antibody fragment or an antibody (e.g., a gene encoding SEQ ID No.: 1 to 4) can be isolated and cloned into one or more expression vectors, and the vector can be transformed into a suitable host cell line for expression of a recombinant polypeptide of the invention. Expression of the gene encoding the polypeptide of the invention provides for increased yield of the polypeptide, and also allows for routine modification of the polypeptide by introducing amino acid substitutions, deletions, additions and other modifications, for example humanizing modifications (Rapley (1995) Mol. Biotechnol. 3: 139-154) in both the variable and constant regions of the antibody fragment or of the antibody of the invention without critical loss of binding specificity or PVR blocking function (Skerra et al. (1993) Curr. Opin. Immunol. 5, 256-262).

Accordingly, in a further embodiment the invention comprises the nucleic acid sequences derived from isolated molecules according to the invention. In still a further embodiment the invention provides the nucleic acid sequence according SEQ ID No.: 5, SEQ ID No.: 6, SEQ ID No.: 71, or SEQ ID No.: 72 as well as homologous DNA sequences or DNA sequences having a degenerated code but still translating into an amino acid sequences having a substantial identity with SEQ ID No.: 1 to 4 or SEQ ID No.: 69 or SEQ ID No.: 70. "Substantial amino acid sequence identity" as used herein means that at least 70%, preferably at least 75%, 80%, 85%, 90%, more preferably all but 5, still more preferably all but 3 and even more preferably all but 1 of the amino acids of two aligned amino acid sequences, particularly of aligned CDRs, are identical.

The present invention further relates in another embodiment to a procaryotic or eucaryotic expression vector comprising a nucleic acid of the invention. The vector is for example a plasmid, a phagemid, or a cosmid. Such expression vectors comprise at least one promotor, at least one signal for translation initiation, at least one nucleic acid sequence of the invention and - in the case of procaryotic expression vectors - a signal for translation termination, while in the case of eucaryotic expression vectors preferably additional signals for transcriptional termination and for polyadenylation.

Examples for prokaryotic expression vectors are, for expression in *Escherichia coli,* e.g. expression vectors based on promoters recognized by T7 RNA polymerase, as described in US 4,952,496, for eucaryotic expression vectors for expression in Saccharomyces cerevisiae, e.g., the vectors p426Met25 or 526GAL1 (Mummberg et al. (1994) Nucl. Acids Res., 22, 5767-5768), for the expression in insect cells, e.g., Baculovirus-vectors as e.g. described in EP-B1-0 127 839 or EP-B1-0 549 721, and for the expression in mammalian cells, e.g., the vectors Rc/CMV and Rc/RSV or SV40-vectors, which are commonly known and commercially available.

The molecular biological methods for the amplification of these expression vectors, as well as the methods of transfecting host cells and culturing such transfected host cells as well as the conditions for producing and obtaining the polypeptides of the invention from said transformed host cells are well known to the skilled person. For example the nucleic acid molecule of the invention can be cloned in a suitable fashion into the expression vectors according to Sambrook et al., (1989) ("Molecular cloning: a laboratory manual" Second edition, Cold Spring Harbor Laboratory Press).

The present invention further relates to a host cell comprising the nucleic acid of the invention and/or the vector of the invention, particularly wherein the host cell is a microorganism like yeast or other fungi, like *Escherichia coli, Bacillus subtilis* or other bacteria. The host cell can also be a cell of higher eucaryotic origin, like an insect cell, preferably a virus infected insect cell, more preferably a baculovirus infected insect cell, or like a mammalian cell like HeLa, COS, MDCK 293-EBNA1, NS0 or a hybridoma cell.

The present invention further provides a method for the production or reproduction of a polypeptide of the invention, particularly an antibody fragment of the invention, comprising culturing a microorganism transformed with a recombinant vector comprising DNA according to the present invention and recovering a relevant polypeptide of the invention, particularly an antibody fragment of the invention or a fusion protein containing it, from the medium.

According to a further embodiment of the invention the expression of a PVR on the surface of a cell can be detected in an assay using a molecule, bioconjugate or polypeptide, antibody or antibody fragment comprising any of the sequences as listed in the sequence list under SEQ ID No.: 1 to 4 or SEQ ID No.: 69 or SEQ ID No.: 70. For this assay a sample is taken from the subject, e.g. a biopsy specimen taken from tissue suspected of having a proliferative disease. Generally, the sample is treated in well-known manner before an assay is performed. Assays, which can be employed, include ELISA, RIA, EIA, Western Blot analysis, immunohistological staining and the like. Depending upon the assay used, the antigens or the antibodies can be labeled by an enzyme, a fluorophore or a radioisotope (See, e.g. Coligan et al. (1994) Current Protocols in Immunology, John Wiley & Sons Inc., New York, New York; and Frye et al. (1987) Oncogene 4, 1153-1157). This assay is particularly useful for identifying patients, which can be susceptible to a treatment with PVR modulating agents, since the cells derived from their primary tumor or their metastasis show a PVR expression.

The present invention further provides a method to modulate and determine the adhesiveness of a naturally occurring cancer cell expressing PVR. This method measures the adhesiveness of a target, e.g. a cell to something else, e.g. another cell, a virus, a complex biological mixture, e.g. extracellular matrix or basal lamina. The method comprises the steps of:
a. contacting the cells with a molecule modulating PVR function;
b. contacting the cancer cell with a layer of ECM proteins, under conditions suitable for the growth of said cancer cells;
c. analyzing the adhesion of said cancer cells to the layer of ECM proteins;
d. optionally, activating an inducible label attached to the molecule and performing step c) again; and
e. determining the percentage of reattachment of cells in step c) and, optionally, step d) in comparison with untreated cells.
The term "layer of ECM proteins" as used herein is understood to be a semi-dry layer of a protein solution, which allows cultivation of cancer cells in contact with the layer and allows attachment of invasive cancer cells to said layer. The thickness of said "layer of ECM proteins" is between 0,1 mm to 1 mm, preferably 0,3 mm thick. Examples for ECM proteins are substances of the extracellular matrix. Particularly ECM proteins are selected from the group consisting of the proteins collagens, entactin, nidogen, vitronectin, fibronectin and laminins. More particularly ECM proteins are selected from collagen S type I, collagen type IV, fibronectin and laminin.

In a preferred embodiment the interfering molecule of step a) is a molecule that specifically binds to an extracellular epitope of PVR, particularly a modified molecule of the invention, more particularly a modified antibody or a modified antibody fragment of the invention, still more particularly a modified antibody fragment, even more particularly a modified scFv, dsFv, Fv, single domain antibody or diabody.

In another embodiment step a) of said method comprises the use of an antisense oligonucleotide against PVR, siRNA or siRNA-like hairpin RNA against PVR or a vector leading to cellular presence of siRNA against PVR or siRNA-like hairpin RNA against PVR. Particularly preferred is the use of siRNA or siRNA-like hairpin RNA against PVR or a vector leading to cellular presence of siRNAs against PVR or siRNA-like hairpin RNA against PVR.

In this particular embodiment of the invention the antisense oligonucleotide moelecule inhibits gene expression of PVR. Preferably, the molecule inhibiting gene expression of PVR can be an antisense oligonucleotide targeted against PVR, an interfering double stranded RNA, commonly known as siRNA (small interfering RNA) targeted against PVR, a siRNA-like hairpin RNAs targeted against PVR function as inhibitors of gene expression, or a vector leading to cellular presence of siRNA targeted against PVR or siRNA-like hairpin RNAs targeted against PVR.

In another preferred embodiment step d) is introduced to the method to activate an inducible label attached to the molecule according to the invention that specifically binds to PVR. If for examples the attached labels are chromophores the an activation via CALI, as described before, may destroy a close-by active domain of the PVR and thereby increase the modulation and/or inhibition of the PVR functionality. A molecule, which previously showed only moderate capability to modulate PVR functionality, e.g., had only neutralizing capacity or even was only binding to the PVR, can thereby become a highly potential modulator or inhibitor of the PVR function(s). The described method allows to quantify the modulation or inhibition of the adhesiveness of cancer cells in comparison with cells bound by non-activated molecules and/or untreated cells.

The adhesive interaction between tumor cells and host cells or the extracellular matrix has been considered as an essential step in the process of metastasis formation. Interference in the adhesive potential of tumor cells with adhesion-blocking agents is therefore a promising approach for preventing metastasis. It is an achievement of the present invention not only to provide molecules binding to the PVR, with maybe neutralizing capacity as described in Lange et al. ((2000) Virology, 285, 218-227), but due to the sophisticated and combined screening, selection and, optional, induction method to identify, isolate and provide molecules which essentially modulate or destroy PVR functions on cells deriving from a proliferative disorder or disease and/or from a naturally occurring cancer. A preferred modulation is an inhibition of a tumor related adhesion behavior, further preferred as modulation is a prevention of a tumor related adhesion behavior by the destruction of active domains of the PVR.

However, an activation or increase of the adhesion behavior e.g. an increase of adhesion behavior to selected immune cells, like e.g. natural killer cells, or to synthetic elements, which could e.g. extract such tumor cells in a blood dialysis, is also provided. In summary, the invention provides molecules or modified or labeled molecules, which actively prevent by binding to or destroying off active domains the tumor related PVR function(s), namely e.g. the adhesive behavior of cancer cells.

The migration of tumor cells into tissue is also an important step in metastasis. The processes of invasion can be studied in the transendothelial model (See, Woodward et al. (2002) Invest Ophthalmol Vis Sci 43, 1708-14 and Vachula et al. (1992) Invasion Metastasis 12, 66-81). A similar transendothelial model provides a further useful *in vitro* assay for the identification of molecules as described above and of screening of molecules modulating or inhibiting the PVR mediated invasion process.

The present invention therefore further provides a method to modulate and determine the invasiveness of a naturally occurring cancer cell expressing PVR. This method comprises the steps of:
a. contacting the cells with a molecule modulating PVR function;
b. contacting the cancer cell with a gel-like matrix, under conditions suitable for the growth of said cancer cells; and
c. analyzing the migration of said cancer cells through the gel-forming matrix;
f. optionally, activating an inducible label attached to the molecule and performing step c) again; and
g. determining the percentage of migration of cells in step c) and, optionally, step d) in comparison with untreated cells.

The term "gel-like matrix" as used herein is understood to be a semi-solid substance with a water content of at least 90%, which allows cultivation of cancer cells in contact with the matrix and allows migration of invasive cancer cells through a slab of said "gel-like matrix" of 0,1 mm to 1 mm, preferably 0,3mm thickness, but not migration of non-invasive cells. Examples for such a "gel-like matrix" are substances resembling the extracellular matrix in protein and carbohydrate composition, particularly the commercially available "Matrigel". Particularly the "gel-like matrix" comprises one of the proteins selected from the group consisting of the proteins collagen type IV, fibronectin and laminin. More particularly the gel-like matrix comprises the proteins collagen type IV, fibronectin and laminin. More preferable the gel-like matrix comprises the proteins collagen type IV, laminin, entactin, nidogen and heparan sulfate proteoglycans or collagen type IV, fibronectin, laminin, nidogen, entactin, and vitronectin.

In a preferred embodiment the interfering molecule of step a) is a molecule that specifically binds to an extracellular epitope of PVR, particularly a modified polypeptide of the invention, more particularly a modified antibody or a modified antibody fragment of the invention, still more particularly a modified antibody fragment, even more particularly a modified scFv, dsFv, Fv, single domain antibody or diabody.

In another preferred embodiment step a) of said method comprises the use of an antisense oligonucleotide against PVR, siRNA or siRNA-like hairpin RNA against PVR or a vector leading to cellular presence of siRNA against PVR or siRNA-like hairpin RNA against PVR as described above.

In another preferred embodiment step d) is introduced to the method to activate an inducible label attached to the molecule according to the invention that specifically binds to PVR. If for examples the attached labels are chromophores the activation via CALI, as described before, may destroy a close-by active domain of the PVR and thereby increase the modulation and/or inhibition of the PVR functionality. A molecule, which previously showed only moderate capability to modulate PVR functionality, e.g. had only neutralizing capacity for one but not another function or even was only binding to the PVR, can thereby become a highly potential modulator or inhibitor of the PVR function(s). The described method allows a quantification of the modulation or inhibition of the invasiveness of cancer cells in comparison with cells bound by non-activated molecules and/or untreated cells. Molecules according to the invention that modulate or inhibit the invasive behavior of cancer cells by more than 20%, more that 40%, more than 60% or even more than 80% are preferred.

The described method, furthermore, allows the selection of the most suitable molecule according to the invention for an individual patient to avoid thereby any individual mismatches, which could interfere with a therapeutic approach.

An angiogenesis assay measures the ability of a cell to induce blood vessel formation, a process, which usually accompanies the growth of malignant tissue. An angiogenesis assay can be performed as described earlier in Kanda et al (2002) J. Natl. Cancer Inst. 94, 1311-9.

The described assays are on one hand side involved in the method to identify suitable molecules according to the invention, which beside binding to the PVR also modulate its function. However, these methods are also highly useful isolated from the method of identifying molecules according to the invention, since they provide a highly selective tool to screen the cancer cells derived from a patient (e.g. by a biopsy) to select suitable molecules according to the invention, which react best with the individual PVR of a patient and thereby show the most effective modulation of the patient derived cancer cells. Preferably, the molecule identified in an *in vitro* assay for a subsequent treatment should inhibit the adhesion, trafficking or invasion behavior of the patient derived tumor cells by about 20, preferably 40%, 50%, 60%, 80% or up to 90%. The subsequent treatment can thereby be specifically tailored and, thus, individual deviations in the receptor binding activities can be avoided.

In another embodiment, the present invention relates to a pharmaceutical composition comprising effective amounts of at least one, but not limited to one molecule according to the invention, particularly a small chemical compound, an antibody, an antibody fragment or bioconjugate modulating the PVR functions or at least one of the molecules inhibiting gene expression of PVR, more particularly wherein the molecule is an antisense oligonucleotide against PVR, an interfering dsRNA (siRNA) or siRNA-like hairpin RNA against PVR or a vector leading to cellular presence of siRNA against PVR or siRNA-like hairpin RNA against PVR, in combination with a pharmaceutically acceptable carrier and/or a diluent. The molecules inhibiting PVR function may also be used as the single effective compound as a medicament or a therapeutic composition.

"Therapeutically effective amounts" are amounts which eliminate or reduce the patient's tumor burden, or which prevent, delay or inhibit metastasis. The dosage will depend on many parameters, including the nature of the tumor, patient history, patient condition, the possible co-use of cytotoxic agents, and methods of administration. Methods of administration include injection (e.g. parenteral, subcutaneous, intravenous, intraperitoreal, etc.), for which the molecule inhibiting PVR function is provided in a nontoxic pharmaceutically acceptable carrier.

"Pharmaceutically acceptable carriers" are, in general, suitable carriers and diluents selected so as not to significantly impair biological activity of the binding agent (e.g. binding specificity, affinity or stability), such as water, saline, Ringer's solution, dextrose solution, 5% human serum albumin, fixed oils, ethyloleate, or liposomes. Acceptable carriers may include biocompatible, inert or bio-absorbable salts, buffering agents, oligo- or polysaccharides, polymers, viscoelastic compound such as hyaluronic acid, viscosity-improving agents, preservatives, and the like. In addition, the pharmaceutical composition or formulation may also include other carriers, adjuvants, or nontoxic, non-therapeutic, non-immunogenic stabilizers, diluents and the like. Typical dosages may range from about 0.01 to about 20 mg/kg, or more particularly from about 1 to about 10 mg/kg.

The pharmaceutical composition can be used for the treatment of conditions related to the over-expression or ectopic expression of human PVR, especially the treatment of tumors, tumors with metastatic potential and/or micro-metastases, especially of metastatic tumors derived from the group selected of cancer cell-types as described above.

In another embodiment, the invention relates to a method of treating or preventing any proliferative disorder or disease, primary or secondary tumors as well as metastasis in a patient comprising the administration of a molecule inhibiting PVR function, in a pharmaceutical acceptable composition in an amount effective to treat, i.e. inhibit, delay or prevent metastasis of PVR mediated adhesion and/or invasion, particularly wherein the molecule inhibits gene expression of PVR, more particularly wherein the molecule is an antisense oligonucleotide against PVR, an interfering dsRNA (siRNA) or siRNA-like hairpin RNA against PVR or a vector leading to cellular presence of siRNA against PVR or siRNA-like hairpin RNA against PVR. Alternatively the molecule inhibiting PVR function can particularly be a molecule which can bind to the extracellular region of PVR, which can be identified by the method of identifying a ligand binding specifically to the extracellular region of PVR, more particularly wherein the molecule is a small chemical compound or an antibody or an antibody fragment or a modified polypeptide of the invention or a bioconjugate of the invention, still more preferably wherein the molecule is a modified scFv of the invention or a modified antibody derived from such a scFv of the invention. Furthermore, the molecule can be labled by chromophores, fluorophores, enzymes or radioisotopes, which can be induced or activated *in vivo* by e.g. the administration of a chemical inducer or the exposition to a photochemical activator. The method of treating or preventing a proliferative disorder, cancer, metastatic tumors, micro-metastases and/or metastasis can be effective to reduce or inhibit the adhesion and/or invasion of naturally occurring cancer cells in particular cancer cells derived from metastatic tumors derived from the group of selected cancer cell-types as described above, since it was shown that blocking PVR function inhibits adhesiveness and/or invasiveness of cancer cells derived from said selected group. In a preferred embodiment, treating of patients according to the present invention, which have naturally occurring cancer cells expressing the PVR with one or more modified antibody fragments causes or leads to a reduction or inhibition of the adhesive and/or invasive ability of human sarcoma cells.

"Treating metastatic tumors" or "treating micro-metastases", as used herein means that cells having a metastatic potential or that metastases of the tumor are stabilized, prevented, delayed, or inhibited by the molecule of the invention, either as a single medicament or in combination with other medicaments. Stable disease or "No Change" (NC) is a description for the course of the disease with either no change of the metastases or a reduction of less than 50% or an increase of less than 25 % over at least 4 weeks. Prevention can be, for example, that no new metastases are detected after the treatment is initiated. This can lead to a two- to three-fold median and/or a 5-year survival rate of treated patients compared with untreated patients. A delay can signify a period of at least 8 weeks, 3 months, 6 months or even one year in which no new metastases are detected after the treatment is initiated. Inhibition can mean that the average size or the total number of new metastases is at least 30%, 40%, 50%, 60%, 70%, 80% or even 90% lower in a group treated with the molecule of the invention in comparison with an untreated group. Number, size and prevalence of metastases can be detected by a skilled practitioner in the field of oncology following generally accepted practice and diagnostic procedures for the detection of metastases, for example as outlined in Harrisons Principles of Internal Medicine 15^{th} ed 2001 Mc Graw Hill.

According to a further embodiment the method of treating according to the invention can be combined with various therapeutic methods. In this context a combination of the treatment with the term "therapeutic methods" means employing the method of treatment with molecules inhibiting PVR function combined with chemotherapy, surgery, and radiation therapy, depending on type of the tumor, patient condition, other health issues, and a variety of factors.

One embodiment of the invention is therefore the use of at least one molecule which can bind to the extracellular region of PVR, which can be identified be the method of identifying a ligand binding specifically to the extracellular region of PVR, more particularly wherein the molecule is a small chemical compound or an antibody or an antibody fragment or a modified polypeptide of the invention or a bioconjugate of the invention, still more preferably wherein the molecule is a modified scFv of the invention or a modified antibody derived from such a scFv of the invention, furthermore, a labeled molecule according to the invention, which can be induced or activated *in vivo* by the administration or application of an inducer for the manufacture of a medicament for the treatment or prevention of adhesion, invasion and/or any metastatic potential of naturally occurring cancer cells, wherein adhesiveness, invasiveness and/or the metastatic potential of said cancer cells is influenced by the PVR function.

The invention therefore provides a method for the production of a pharmaceutical composition or medicament comprising the step of incorporating the molecule or the ligand according to the present invention into a therapeutic, prophylactic or diagnostic composition. This can, e.g., be done by mixing the identified ligand or an modified ligand with a pharmaceutically acceptable carrier known in the art, wherein the ligand is present in an amount, which is therapeutically effective.

In another embodiment, the present invention encompasses a diagnostic kit. Such a kit comprises at least one bioconjugate and/or at least one labeled molecule or polypeptide of the invention and/or an antibody fragment or an antibody of the invention, or a labeled version of these, and consists additionally of the reagents and materials necessary to carry out a standard competition or sandwich assay. Said diagnostic kit may be used for the determination of the invasive potential of biological samples, in particular of certain cancer cell types. A kit will further typically comprise a container.

The following examples, including experiments conducted and results achieved, are provided for illustrative purposes only and are not to be construed as limiting upon the present invention.

### DESCRIPTION OF THE DRAWINGS

- Figure **1**: shows the invasion of stained HT1080 cells through a Matrigel coated 8µm filter. Fluorescence was quantified after six hours incubation at 37°C. Data presented are the mean of n = 3 wells +/- SD.
- Figure **2a**: shows the inhibitory effect of scFv1 on the invasion of HT1080 cells. Invasion was determined in a chemotaxis assay with a Matrigel coated cell migration chamber. The invasion was determined after laser irradiation (with CALI). The invasion of HT1080 cells in the absence of any inhibitory molecule was used as a control (left bar). The molecule scFv1 inhibited the invasion of HT1080 cells by about 22% (p-value < 0.05)
- Figure **2b**: shows the inhibitory effect of a commercial anti PVR antibody (D171), scFv3 and scFv4 on the invasion of HT1080 cells. Invasion was determined in a chemotaxis assay with a Matrigel coated cell migration chamber. The invasion was determined before and after light irradiation (black bars without CALI, grey bars with CALI). The invasion of HT1080 cells in the absence of any inhibitory molecule (0) was used as a control (left bars). The molecule scFv3 and scFv4 inhibited the invasion of HT1080 cells by about 30% (p-value < 0.001)
- Figure **2c**: shows the inhibitory effect of scFv3 and scFv4 on the invasion of MDA-MD231 cells. Invasion was determined in a chemotaxis assay with a Matrigel coated cell migration chamber. The invasion was determined before and after light irradiation (black bars without CALI, grey bars with CALI). The invasion of HT1080 cells in the absence of any inhibitory molecule (0) was used as a control (left bars). scFv3 and scFv4 inhibited the invasion of MDA-MD231 cells by about 23% (p-value < 0.001)
- Figure **3**: shows the inhibitory effect of scFv1 and scFv2 on the adhesion of HT1080 cells to collagen S type I. The adhesion was determined after laser irradiation (with CALI). The adhesion of HT1080 cells in the absence of any inhibitory molecule was used as a control (left bar). The molecule scFv1 inhibited the adhesion by about 56% and the molecule scFv2 by 42% (p-value < 0.05).
- Figure **4**: shows a result of FACS analysis. The binding activity of scFv1 and scFv2 to HT1080 cells (bold line) and to HS-27 cells (dotted line) as control is illustrated. Also the binding of scFv1 to PC3-cells, KHOS-cells and Liver cells is illustrated.
- Figure **5**: shows the result of the immunoprecipitation experiments. scFv1 and scFv2 were incubated with lysates of HT1080 cells and of HS-27 cells as a control. The immuno-complexes were separated by SDS-PAGE and silver stained. The scFv band and also the PVR specific band are indicated.
- Figure **6**: shows a MALDI-MS spectrum of the peptide mixture obtained from the band with an approximate size of 75 kDa immunoprecipitated with scFv 1. Two trypsin auto digestion peaks, indicated as T, were used for internal calibration. A total of 6 peaks, marked with asterisks, matched PVR (SwissProt, P15151), with a mass deviation of less than 10 ppm. The matched peptides cover 18 % (74/417 residues) of the protein.

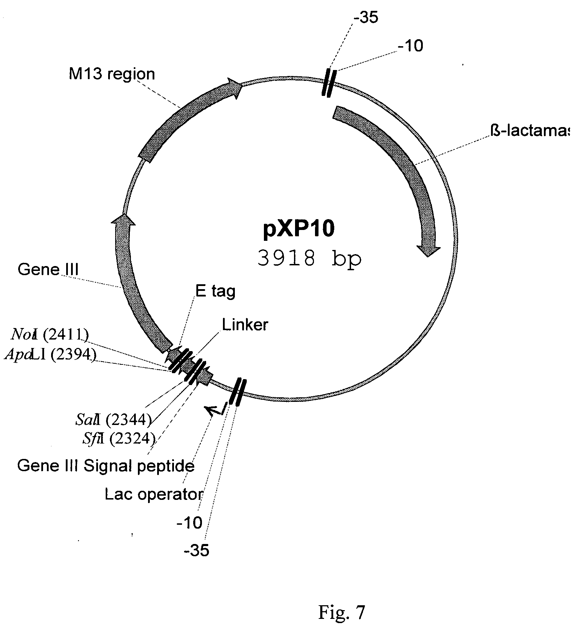

- Figure **7**: shows the vector map of the scFv display vector pXP10 and corresponding sequence (SEQ ID No.: 7)

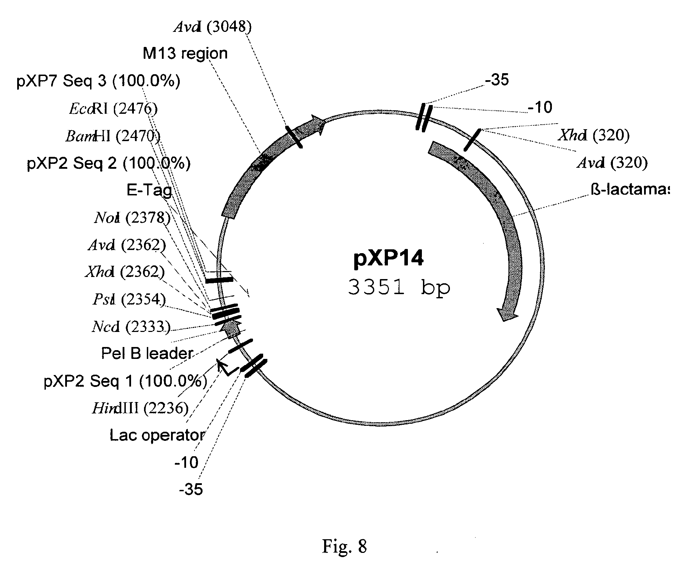

- Figure **8**: shows the vector map of the scFv expression vector pXP14 and corresponding sequence (SEQ ID NO.: 8)

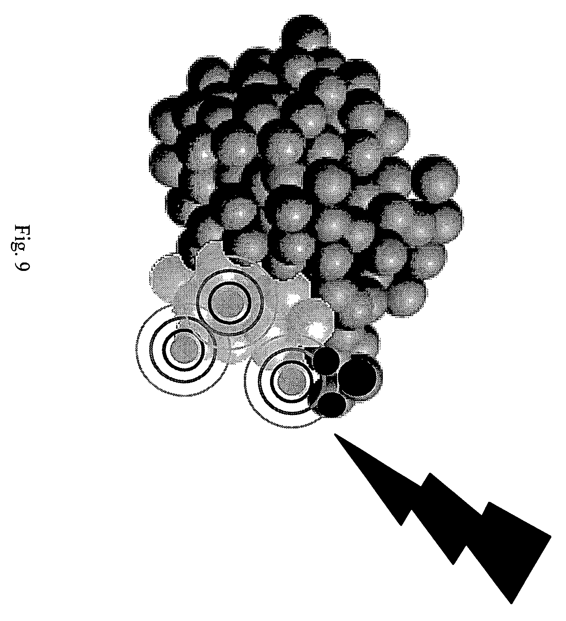

- Figure **9**: shows the amino acid sequences (SEQ ID NO.: 3) and nucleotide sequence (SEQ ID NO.: 5) of scFv1
- Figure **10**: shows the amino acid sequences (SEQ ID NO.: 4) and nucleotide sequence (SEQ ID NO.: 6) of scFv2
- Figure **11**: shows the amino acid sequences (SEQ ID NO.: 69) and nucleotide sequence (SEQ ID NO.: 71) of scFv3
- Figure **12**: shows the amino acid sequences (SEQ ID NO.: 70) and nucleotide sequence (SEQ ID NO.: 72) of scFv4
- Figure **13**: the principle of Chromophore Assisted Laser Inactivation (CALI)
- Figure **14**: Sequences of the primers SEQ ID No.: 9 to 52, 63 to 68.

### EXAMPLES

### Example 1:

### Construction of an immune library

Two BALB/c mice were each immunized intradermally with 2 x 10⁷ paraformaldehyde fixed HT1080 cells (human fibrosarcoma cell line; ATCC, CCL-121). Following the first immunization, the injections were repeated twice in a period of 39 days, the mice sacrificed and the spleens isolated and frozen in liquid nitrogen.

Total RNA was isolated using the RNeasy Midi Kit (QIAGEN #75142) as described by the manufacturer using half of each spleen preparation. The RNA concentration and purity was determined by a denaturing formaldehyde gel and photometric measurement.
cDNA was synthesized using 8.9 µg of freshly prepared RNA and 10 pmol of a primer mix (IgG1-c (SEQ ID NO.: 63), IgG2a-c (SEQ ID NO.: 64), IgG2b-c (SEQ ID NO.: 65), IgG3-c (SEQ ID NO.: 66), VLL-c (SEQ ID NO.: 67), VLK-c (SEQ ID NO.: 68)) using the Superscript™ II Kit (GibcoBRL Life Technologies #18064-014). These primers anneal to the RNA encoding the IgG heavy-chain (VH) genes and the light chain (VL) genes of the kappa and lambda family. VH genes were PCR amplified from 1 µl of cDNA using 36 individual combinations of 9 forward primers (M-VH1 (SEQ ID NO.: 9), M-VH2 (SEQ ID NO.: 10), M-VH3 (SEQ ID NO.: 11), M-VH4 (SEQ ID NO.: 12), M-VH5 (SEQ ID NO.: 13), M-VH6 (SEQ ID NO.: 14), M-VH7 (SEQ ID NO.: 15), M-VH8 (SEQ ID NO.: 16), M-VH9 (SEQ ID NO.: 17)) and 4 backward primers (M-JH1 (SEQ ID NO.: 18), M-JH2 (SEQ ID NO.: 19), M-JH3 (SEQ ID NO.: 20), M-JH4 (SEQ ID NO.: 21)) without restriction sites. VL genes were PCR amplified with one primer mix (M-VK1 (SEQ ID NO.: 22), M-VK2 (SEQ ID NO.: 23), M-VK3 (SEQ ID NO.: 24), M-VK4 (SEQ ID NO.: 25), M-VL1 (SEQ ID NO.: 26), M-JK1 (SEQ ID NO.: 27), M-JK2 (SEQ ID NO.: 28), M-JK3 (SEQ ID NO.: 29), M-JL1 (SEQ ID NO.: 30)) without restriction sites. PCR products were gel-purified (QIAquick Gel Extraction Kit, #28706) and reamplified using individual combinations of 9 forward primers (MVH1 SfiI (SEQ ID NO.: 31), MVH2 SfiI (SEQ ID NO.: 32), MVH3 SfiI (SEQ ID NO.: 33), MVH4 SfiI (SEQ ID NO.: 34), MVH5 SfiI (SEQ ID NO.: 35), MVH6 SfiI (SEQ ID NO.: 36), MVH7 SfiI (SEQ ID NO.: 37), MVH8 SfiI (SEQ ID NO.: 38), MVH9 SfiI (SEQ ID NO.: 39)) and 4 backward primers (M-JH1 SaIl (SEQ ID NO.: 40), M-JH2 SalI (SEQ ID NO.: 41), M-JH3 SalI (SEQ ID NO.: 42), M-JH4 SalI (SEQ ID NO.: 43)) with restriction sites for VH and one primer mix (M-VK1 ApaLI (SEQ ID NO.: 44), M-VK2 ApaLI (SEQ ID NO.: 45), M-VK3 ApaLI (SEQ ID NO.: 46), M-VK4 ApaLI (SEQ ID NO.: 47), M-VL1 ApaLI (SEQ ID NO.: 48), M-JK1 NotI (SEQ ID NO.: 49), M-JK2 NotI (SEQ ID NO.: 50), M-JK3 NotI (SEQ ID NO.: 51), M-JL1 NotI (SEQ ID NO.: 52)) with restriction sites for VL. PCR products were gel-purified (QIAquick Gel Extraction Kit, #28706) and cloned into the phage display vector pXP10 (SEQ ID No.: 7) using the restriction sites *SfiI*/*SalI* for VH and *ApaLI*/*NotI* for VL. The ligation mix was transfected into *E.coli* TG-1 by electroporation resulting in a library size of 10⁷ independent clones (phages) expressing different single chain antibody fragments (scFv).

### Example 2:

### Selection of tumor cell specific scFv (Selection on fixed cells)

Phages expressing scFv with high affinity to tumor cells were selected as follows: HT1080 cells were harvested with 0.05% EDTA, fixed with paraformaldehyde, diluted to 1x10⁷ cells/ml in PBS and immobilized onto wells of a 96 well UV cross-link plate (Corning Costar). The wells of the UV cross-link plate were blocked with 5% Skim Milk Powder (#70166, Fluka) in PBS (MPBS). 10¹² cfu (colony forming units) of phage library/10⁶ cells were pre-blocked for 1 hour at 25°C with MPBS and subsequently incubated for 1.5 hour at room temperature (RT) with the cells. The wells of the UV cross-link plate were washed six times with PBS + 0.05% Tween-20 followed by six washes with PBS. Bound phage were eluted by the addition of 10 mM Glycine pH 2.2, and neutralized with 1 M Tris/HCl pH 7.4. Typically, between 10³ and 10⁶ cfu were eluted in the 1^{st} round of selection, thus the diversity of the enriched repertoire is decreased compared to the original repertoire. The eluate containing the enriched repertoire was amplified by infecting exponentially growing *E. coli* TG1. Phagemid containing *E. coli* were selected and propagated by overnight (o/n) growth at 30°C on LB agar plates supplemented with 100 µg/ml ampicillin and 1% glucose. Following this step, the enriched repertoire can either be amplified as a polyclonal pool and used for further rounds of selection in an iterative manner until convergence to desired properties is achieved or be spatially separated and screened on a single clone level. Phage particles for the next round of selection were produced by super-infecting exponentially growing cultures of the previous round of selection with helper phage VCS-M13 (Stratagene, La Jolla, CA) and growing the cultures overnight at 20°C in 2xTY supplemented with 100 µg/ml ampicillin (amp) and 50 µg/ml kanamycin (kan). Selection ready phage were precipitated with 0.5 M NaCl/4% PEG-6000 from the cleared bacterial supernatant and re-suspended in PBS. One round of selection was performed, followed by screening on a single clone level as described in example 3.

### Example 3:

### Screening of scFv (Screening on fixed cells)

For screening, the genes encoding the selected scFv, contained in the phage display vector, were re-cloned to the expression vector pXP14 (SEQ ID No.: 8). This vector directs the expression of scFv in fusion with a Strep-tag and E-tag and does not contain a filamentous phage gene-3. Expression vector containing *E. coli* TG1 from single colonies were grown in individual wells of a micro titer plate so that each well contains only one scFv clone. The bacteria were grown at 30°C in 2xTY supplemented with 100 µg/ml ampicillin and 0.1% glucose in 96-well micro titer plates (#9297, TPP) until an OD₆₀₀ of 0.7. Expression was induced with IPTG at a final concentration of 0.5 mM and continued at 25°C overnight. Single chain Fv containing cleared lysates were prepared by addition of hen-egg lysozyme (#L-6876, Sigma) to a final concentration of 50 µg/ml for 1 hour at 25°C and centrifugation for 15 minutes at 3000 x g. Prior to the screening ELISA, the cleared lysates were blocked by the addition of an equal volume of DMEM + 10% FCS for 1 hour. For the screening ELISA, HT1080 cells were harvested with 0.05% EDTA, fixed with paraformaldehyde, diluted to 1x10⁷cells/ml in PBS and immobilized onto wells of a 96 well UV cross-link plate (Coming Costar). The wells of the UV cross-link plate were blocked with MPBS and the scFv containing blocked cleared lysates added for 1.5 hours at 25°C. The plates were washed 2x with PBS + 0.1% Tween-20 and 1x with PBS, incubated with HRP conjugated α-E-tag (#27-9413-01, Pharmacia Biotech; diluted 1:5000 in MPBS with 0.1 % Tween-20) for 1 hour, washed 3x with PBS + 0.1% Tween-20 and 3x with PBS, developed with POD (#1 484 281, Roche) and signals read at 370 nm.

Positive clones were retested against HT1080 cells and control human fibroblasts Hs-27 (ATCC CRL-1634) using the ELISA screening procedure described above and preserved in glycerol stocks. In a typical screen, 2760 (30x92) clones were screened for binding to HT1080 cells with 5 % positives defined as clones giving a background subtracted signal > 0.1. 155 positive clones were retested for specific binding to HT1080 cells compared to the Hs-27 control cells with 28 % positives defined as clones giving a background subtracted signal on HT1080 of at least twice the value of the signal on Hs-27 control cells.

### Example 4:

### Sequencing and large scale expression of identified scFv

Sequencing of scFv1-scFv4 and their genes was performed by Sequiserve GmbH, Vaterstetten, Germany using the primer pXP2 Seq2 (5'-CCCCACGCGGTTCCAGC-3' (SEQ ID No.: 53) and pXP2 Seq1 (5'TACCTATTGCCTACGGC-3' (SEQ ID No.: 54). The amino acid sequence and nucleotide sequence are shown in the Figures.
Unique clones identified by sequencing were streaked out from glycerol stocks onto LB/Amp(100µg/ml)/1% Glucose Agar plates and incubated o/n at 30°C. 10 ml LB/Amp/Glu (1%) media were inoculated with a single colony and grown o/n at 30°C and 200 rpm shaking. The next morning the overnight cultures were placed on ice until inoculation of 1L 2xTY media supplemented with 100µg/ml Ampicillin and 0.1% Glucose in 2L Erlenmeyer-flasks. The cultures were grown at 25°C shaking until an OD₆₀₀ 0.5 - 0.6 was reached and then induced with IPTG 0.1 mM final concentration. Fresh ampicillin was added to 50 µg/ml and incubation was proceeded at 22°C o/n shaking. In the morning the cultures were centrifuged at 5000 x g for 15 minutes at 4°C, supernatants discarded and the pellets re-suspended carefully on ice with a pipette in 10 ml pre-cooled PBS-0.5 M Na buffer containing protease inhibitors complete (#1697498, Roche). After re-suspension was completed, bacterial suspensions were transferred to 20 ml oakridge centrifuge tubes and hen-egg lysozyme (#L-6876, Sigma) added to a final concentration of 50 µg/ml for 1 hour on ice. The lysed bacteria were centrifuged at 20000 x g for 15 minutes at 4°C and the supernatants (lysate) transferred to a 15 ml plastic tube. For affinity purification the lysates were loaded with 1 ml/min onto 1ml StrepTactin (# 2-1505-010, IBA) columns equilibrated with 10 column volumes (CV) PBS-0.5 M Na buffer via a parallel protein purification system. After a 10 CV wash with PBS the elution was done with 5 CV PBS/5mM Desthiobiotin (#D-1411, Sigma) and 1 ml fractions collected. The fractions were measured at UV₂₈₀, protein containing fractions were pooled and concentrated with Amicon Ultra Centrifugal Filter Devices 10.000 MWCO (#UFC801024, Millipore) at 4700 x g.
The concentrated scFv were checked on 12% Bis-Tris SDS-PAGE gels stained with Coomassie Blue for purity and frozen in aliquots with 20 % glycerol at -80°C.

### Example 5:

### FACS analysis for tumor cell specific binding

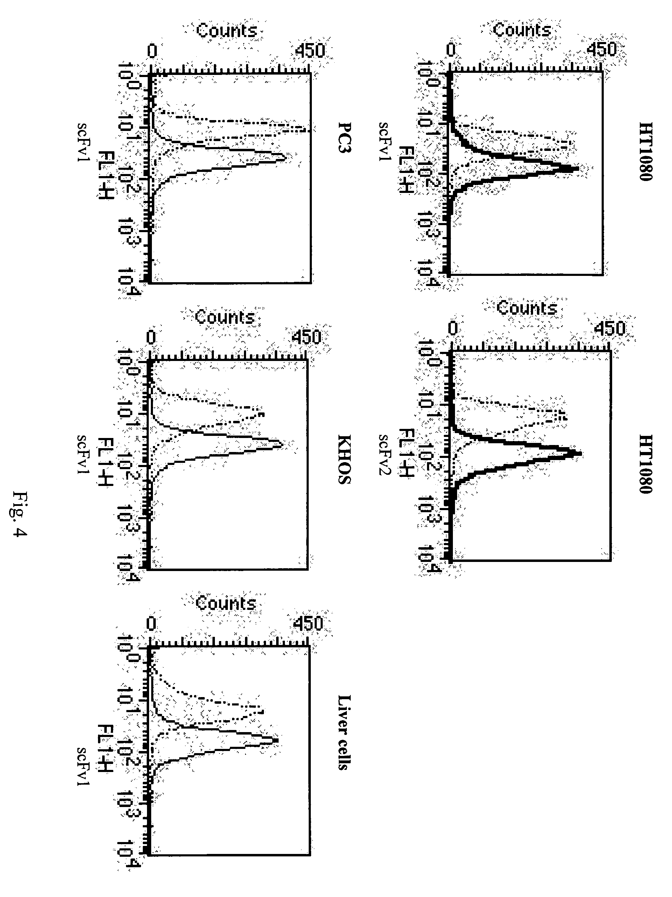

To test the ability of purified anti HT1080 scFv to bind specifically to target cells, we performed a fluorescence-activated cell sorter (FACS) analysis using HT1080 cells (ATCC CCL-121) and Hs-27 cells (10⁶ cells/ml) as control cell line (see Figure 4). ScFv were also tested for binding to KHOS cells (ATCC CRL-1544), PC-3 cells (ATCC CRL-1435), and chang liver cells (DSMZ ACC-57 contain impurities of Hela cells) (for all 10⁶ cells/ml) compared to HT1080 cells. Cells were incubated with 10 µg/ml of pure scFv in CellWash (BD (Becton, Dickinson and Company) #349524) for 20 min at 4°C, washed, and bound scFv's were detected with a secondary FITC labeled anti E-tag mab (Amersham #27-9412-01). Samples were washed and analyzed on a Becton Dickinson FACSscan. Figure 4 (upper panel) shows the log fluorescence intensity (FLI-H; x-axis) versus the relative cell numbers (counts; y-axis) for cells reacting with scFv1 and scFv2. The thin line represents the control cell line (HS-27) and the bold line the HT1080 cells. scFv1 and scFv2 specifically stain the tumor cell lines with up to 10 fold higher signals compared to the control cell line. The lower panel of Figure 4 shows the binding of scFv1 to PC3, KHOS and chang liver cells compared to HT1080 cells. scFv1 binds to all three cells lines, but to a lower degree compared to HT1080 cells (PC3, KHOS or chang liver cells = dotted line).

### Example 6:

### Competition analysis by FACS

To test the ability of the purified anti-PVR-scFv to block common PVR epitopes on the target cells, single cell suspensions of HT1080 are harvested with 0,5 mM EDTA/PBS. Approximately 1x10⁶ cells are incubated in CellWash (BD, #349524) with 10 µg/ml scFv for one hour at 4°C. After washing with Cell Wash 10µg/ml FITC labeled scFv is added and incubated for 20 min at 4°C. Signals of bound FITC labeled scFvs with and without pre-incubation of other PVR binders are analyzed on a Becton Dickinson FACSscan.

### Example 7:

### Labeling of antibody fragments with FITC

scFv were labeled with fluorescein isothiocyante (FITC) (Molecular Probes, Eugene, USA #F1906) by the following method: Aliquots of a 10mg/ml solution of FITC in dimethyl sulfoxide were added to 100µg of scFv dissolved in PBS/0.5M NaHCO₃, pH 9.5 in a ratio of 30:1 (FITC:scFv1). The sample was incubated for two hours at room temperature with agitation, free FITC was separated using desalting columns (2 Micro Spin G-25, Pharmacia 27-5325-01). The ratio of labeling was determined via mass spectrometry and via UV/VIS spectroscopy, whereby the protein concentration was calculated at 280nm and the FITC concentration at 494nm.

### Example 8:

### Invasion assay for identification of inhibitory molecules

The ChemoTx® system (Neuro Probe Inc.#106-8, Gaithersburg) may be used as a disposable chemotaxis/cell migration chamber in a 96 well format with an 8 µm filter Track etched Polycarbonate pore size, 5.7 mm diameter/site.

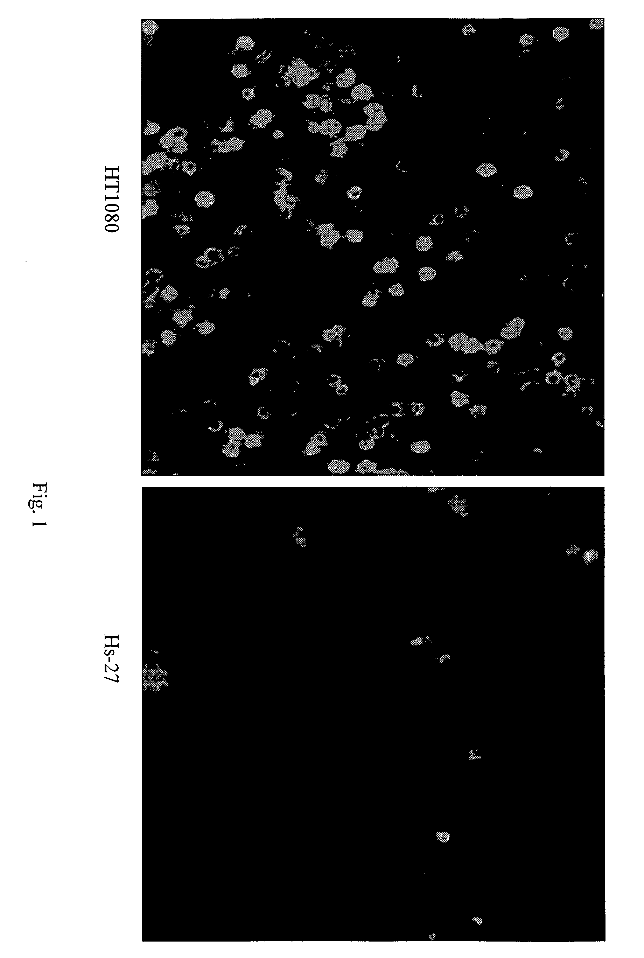

13,3 µl of 0.3 mg/ml Matrigel (Matrigel is a solubilized basement membrane preparation extracted from the Engelbreth-Holm-Swarm (EHS) mouse sarcoma, a tumor rich in extracellular matrix proteins. Its major component is laminin, followed by collagen IV, heparan sulfate proteoglycan, entactin and nidogen. It also contains TGF-alpha fibroblast growth factor, tissue plasminogen activator, and other growth factors which occur naturally in the EHS tumor) (Becton Dickenson, BD #356234) diluted in Dulbeccos PBS (Gibco #14040-091) is applied on the membrane filter of the 96-well plate on row B-H and on row A 1,2 µg/site of collagen S type I (Roche #10982929) is diluted in 0,05 M HCl (Sigma #945-50) and is incubated over night at 20°C in a desiccator for gelation. HT1080 cells are grown to 70-80% confluence in DMEM supplemented with GlutamaxI (862mg/l (Gibco #31966-021) with 10% FCS (Gibco #10270106). The cells are washed 2x with DMEM/GlutamaxI/0.1 % BSA (Sigma #A-7030) then labeled *in situ* with Bisbenzimide H 33342 (Sigma #B-2261) are diluted 1:100 in DMEM/GlutamaxI/0.1 % BSA for 15 min at 37°C, 7,5% CO₂. Cells are washed 2x with DMEM/GlutamaxI/0.1 % BSA and are loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells are detached with 0.5mM EDTA (Sigma #E8008), are collected with Dulbeccos PBS/0.1% BSA/10mM Hepes (Gibco #15630-056), are washed 2x with Dulbeccos PBS/0.1% BSA/10mM Hepes, are suspended in Dulbeccos PBS/0.1% BSA/10mM Hepes and are diluted to 6,7 x 10⁶ cells/ml with Dulbeccos PBS/0.1% BSA/10mM Hepes. 6,7 x 10⁶ cells/ml are incubated 1:1 with 40µg/ml of a control scFv as a negative control for inhibition of invasion and with HT1080 specific scFv for 1h on ice. After dilution to 6,7 x 10⁵ cells/ml with DMEM/GlutamaxI/0.1 % BSA, HT1080 cells and HT1080 cell/scFv dilutions are pipetted in triplicate onto the chemotaxis chamber (row B-H) at a density of 3,4 x 10⁴ cells/well and are incubated for 6 h at 37°C, 7,5% CO₂. DMEM/GlutamaxI with 5% FCS is used as a chemo attractant in the lower chamber. A standard curve from 1x10⁴ to 4x10⁴ cells/site is performed on collagen S type I coated row A of the chemotaxis chamber. DMEM/GlutamaxI/0.1% BSA is used in the lower chamber (cells are not migrating). After scraping the non-migrating cells from the top of the membrane (except the Standard curve on row A) fluorescence of cells, which had migrated through the membrane (not migrated in case of the Standard curve), is measured on the Fluostar Galaxy (bMG) microplate reader using excitation/emission wavelengths of 370/460 nm. (See, Figure 1).

### Example 9:

### Invasion assay for target identification with CALI

This Example is in principle identical to Example 8, except for the use of FITC-labeled scFv and the additional integration of the CALI process within the invasion assay. In the context of the CALI process the scFv was labeled with a CALI inducible label.

The ChemoTx® system (Neuro Probe Inc.#106-8, Gaithersburg) may be used as a disposable chemotaxis/cell migration chamber in a 96 well format with an 8 µm filter Track etched Polycarbonate pore size, 5.7 mm diameter/site. 13,3 µl of 0.3 mg/ml Matrigel (see Example 8) diluted in Dulbeccos PBS (Gibco #14040-091) is applied on the membrane filter of the 96-well plate on row B-H and on row A 1,2 µg/site of collagen S type I (Roche #10982929) is diluted in 0,05 M HCl (Sigma #945-50) and is incubated over night at 20°C in a desiccator for gelation. HT1080 cells were grown to 70-80% confluence in DMEM supplemented with GlutamaxI (862mg/l (Gibco #31966-021) with 10% FCS (Gibco #10270106). The cells were washed 2x with DMEM/GlutamaxI/0.1 % BSA (Sigma #A-7030) then labeled *in situ* with Bisbenzimide H 33342 (Sigma #B-2261) and diluted 1:100 in DMEM/GlutamaxI/0.1 % BSA for 15 min at 37°C, 7,5% CO₂. Cells were washed 2x with DMEM/GlutamaxI/0.1 % BSA and loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells were detached with 0.5 mM EDTA (Sigma #E8008), collected with Dulbeccos PBS/0.1% BSA/10 mM Hepes (Gibco #15630-056), washed 2x with Dulbeccos PBS/0.1% BSA/10 mM Hepes, suspended in Dulbeccos PBS/0.1% BSA/10 mM Hepes and diluted to 6,7 x 10⁶ cells/ml with Dulbeccos PBS/0.1% BSA/10mM Hepes. 6,7 x 10⁶ cells/ml are incubated 1:1 with 40 µg/ml of FITC-labelled antibetal integrin monoclonal antibody (JB1, Chemicon #MAB1963) as a control for inhibition of invasion after CALI and with HT1080 specific FITC labelled scFv for 1h on ice. 1,3x10⁵ HT1080 cells/well or HT1080 cell/scFv or Ab dilution were pipetted in triplicate in two 96-well plate, black, ultra thin clear flat bottom special optics (Costar #3615). One plate was kept on ice in the dark while the other plate was irradiated on an ice block with continuous wave laser at 488 nm (0.5W, 30 sec). After dilution to 6,7 x 10⁵ cells/ml with DMEM/GlutamaxI/0.1 % BSA, HT1080 cells and HT1080 cell/scFv dilutions were pipetted in triplicate (non irradiated triplicate beside irradiated triplicate) onto the chemotaxis chamber (row B-H) at a density of 3,4 x 10⁴ cells/well and incubated for 6 h at 37°C, 7,5% CO₂. DMEM/GlutamaxI with 5% FCS is used as a chemo attractant in the lower chamber. A standard curve from 1x10⁴ to 4x10⁴ cells/site was performed on collagen S type I coated row A of the chemotaxis chamber. DMEM/GlutamaxI/0.1% BSA was used in the lower chamber (cells are not migrating). After scraping the non-migrating cells from the top of the membrane (except the Standard curve on row A) fluorescence of cells, which had migrated through the membrane (not migrated in case of the Standard curve), was measured on the Fluostar Galaxy (bMG) microplate reader using excitation/emission wavelengths of 370/460nm. The result of the invasion assay with laser irradiation is shown in Figure 2. scFv1 inhibits the invasion by about 22%.

### Example 9.1:

### Invasion assay for target identification with CALI (with blue-filtered light)

This Example is in principle identical to Example 9, except for the irradiation of samples with blue-filtered 300 W light instead of irradiation with a laser.

HT-1080 cells were incubated with 20_g/mL of FITC-labeled antibody for 1 hour, then irradiated with blue filtered 300Watt light for another hour. The cells were then assayed for invasiveness through a Matrigel coated porous membrane (Neuro Probe Inc.) for 6 hours. Bars represent percent invasion normalized to no irradiation conditions ± standard error, and represent at least two independent experiments in triplicate. The left bar in Fig. 2b (0) shows the invasion of cells that were not treated with any antibody, D171 (NeoMarker # MS-465) is a commercially available monoclonal antibody directed against PVR/CD155. Both scFv, scFv3 and scFv4, inhibited the invasion of HT1080 cells by about 30%.

### Example 9.2:

### Invasion assay with breast cancer cells

This Example is in principle identical to Example 9.1, except for the use of MDA-MD231 breast cancer cells instead of HT1080 cells. Both scFv, scFv3 and scFv4, showed an inhibition of invasion of MDA-MD231 cells by about 23%.

### Example 10:

### MTS viability assay

Viable cells were detected by measuring the conversion of the tetrazolium dye MTS (MTS, Celltiter A_{queous} one, Promega #G4000) to formazan. HT1080 cells and HT1080 cell/scFv dilutions (obtained from the dilutions prepared in the Adhesion assay) were pipetted in triplicate at a density of 3,4 x 10⁴ cells/well and were plated in a 96-well plate (black, ultra thin clear flat bottom, special optics, Costar #3615). 10 µl MTS was added to each well and incubated for 1 hour at 37°C, 7,5% CO₂. Absorbance was measured at 492 nm with the Fluostar Galaxy (bMG) microplate reader. For the tested scFv1 and scFv2, no effect on viability of cells was seen.

### Example 11:

### Cell-matrix adhesion assay for identification of inhibitory antibody fragments

21 wells of a 96-well flat bottom plate (Costar #3614) are coated with one matrix protein selected from collagen S type I 1 µg/well (Roche #10982929), collagen type IV 1 µg/well (Rockland 009-001-106), fibronectin 1µg/well (Sigma F2518) and laminin 1 µg/well (Roche 1243217) in Dulbeccos PBS (Gibco #14040-091), respectively, at 4° C over night. At the same time 3 wells in row A are coated with 2% BSA (Sigma #A-7030)/Dulbeccos PBS for a blank value. Wells are washed twice with Dulbeccos PBS, and blocked with 2% BSA (Sigma #A-7030)/Dulbeccos PBS for 1h at 37°C and washed with Dulbeccos PBS. HT1080 are harvested, stained with 2,5 mM (final concentration) Calcein AM (Molecular Probes C-3099), washed twice with PBS w/o CaCl₂ w/o MgCl₂ (Gibco 10010-015) and diluted to 1,5 x 10⁵/ml in buffer (0,5% BSA (Sigma #A-7030) + 10mM Hepes + DMEM (Gibco 31966-02)). HT1080 cells are mixed with 10µg/ml scFv and incubated for 30 min on ice. The HT1080 cells alone and HT1080/scFv dilutions are pipetted in triplicate at a density of 1,5x10⁴ cells/well and incubated for one hour at 37°C, 7,5% CO₂. After two additional washing steps with Dulbeccos PBS, where non-adherent cells are washed away, a Standard curve from 3,7 x 10³ to 1,5 x 10⁴ stained cells/well diluted in Dulbeccos PBS may be performed in triplicate in row A. Washed wells are filled with 100µl Dulbeccos PBS and the absorbance of attached cells and of the Standard curve is measured on the Fluostar Galaxy (bMG) microplate reader using excitation/emission wavelengths of 485/520 nm.

### Example 12:

### Cell-matrix adhesion assay for target identification with CALI

96-well plates (TPP #9296) (cell culture treated) were coated in Row B-H with collagen S type I 1 µg/well (Roche #10982929) in Dulbeccos PBS (Gibco #14040-091) and in Row A well 10-12 were coated with 2% BSA (Sigma #A-7030)/Dulbeccos PBS at 4°C over night. The plate was washed with Dulbeccos PBS, blocked Row B-H and Row A well 10-12 with 2% BSA/Dulbeccos PBS for 1h at 37°C and washed again with Dulbeccos PBS. HT1080 cells were grown to 70-80% confluence in DMEM supplemented with GlutamaxI (862 mg/l (Gibco #31966-021) with 10% FCS (Gibco #10270106). The cells were washed 2x with DMEM/GlutamaxI/0.1 % BSA (Sigma #A-7030) then labeled *in situ* with Bisbenzimide H 33342 (Sigma #B-2261) were diluted 1:100 in DMEM/GlutamaxI/0.1 % BSA for 15 min at 37°C, 7,5% CO₂. Cells were washed 2x with DMEM/GlutamaxI/0.1 % BSA and loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells were detached with 0.5 mM EDTA (Sigma #E8008), collected with Dulbeccos PBS/0.1% BSA/10mM Hepes (Gibco #15630-056), washed 2x with Dulbeccos PBS/0.1% BSA/10mM Hepes, suspended in Dulbeccos PBS/0.1% BSA/10mM Hepes and diluted to 6,7 x 10⁶ cells/ml with Dulbeccos PBS/0.1% BSA/10mM Hepes. 6,7 x 10⁶ cells/ml were incubated 1:1 with 40 µg/ml of FITC-labeled anti-beta1 integrin monoclonal antibody (JB1, Chemicon #MAB1963) as a control for inhibition of adhesion after CALI and with HT1080 specific FITC labeled scFv for 1h on ice. 1,3x10⁵ HT1080 cells/well or HT1080 cell/scFv or Ab dilution were pipetted in triplicate in two 96-well plate, black, ultra thin clear flat bottom special optics (Costar #3615). One plate was kept on ice in the dark while the other plate was irradiated on an ice block with continuous wave laser at 488 nm (0.5W, 30 sec). After dilution to 6,7 x 10⁵ cells/ml with DMEM/GlutamaxI/0.1 % BSA, HT1080 cells and HT1080 cell/scFv dilutions were pipetted in triplicate (non irraditated triplicate beside irradiated triplicate) onto the coated and blocked plate. In Row A well 10-12 6,7 x 10⁵ cells/ml with DMEM/GlutamaxI/0.1 % BSA were pipetted as a background control. Plate was incubated for 1h at 37°C, 7,5% CO₂ and washed 2x with Dulbeccos PBS, where non-adherent cells were washed away. In Row A well 1-9 a standard curve from 1x10⁴ to 4x10⁴ cells/well was performed, in all other wells 50 µl Dulbeccos PBS was pipetted. Fluorescence of cells, which had adhered to the Collagen S type 1 (not adhered in case of the Standard curve), was measured on the Fluostar Galaxy (bMG) microplate reader using excitation/emission wavelengths of 370/460 nm. scFv1 inhibited the adhesion by 55.5% and scFv2 inhibited the adhesion by 42%. Results for both scFv's are shown in Figure 3.

### Example 13:

### Immunoprecipitation

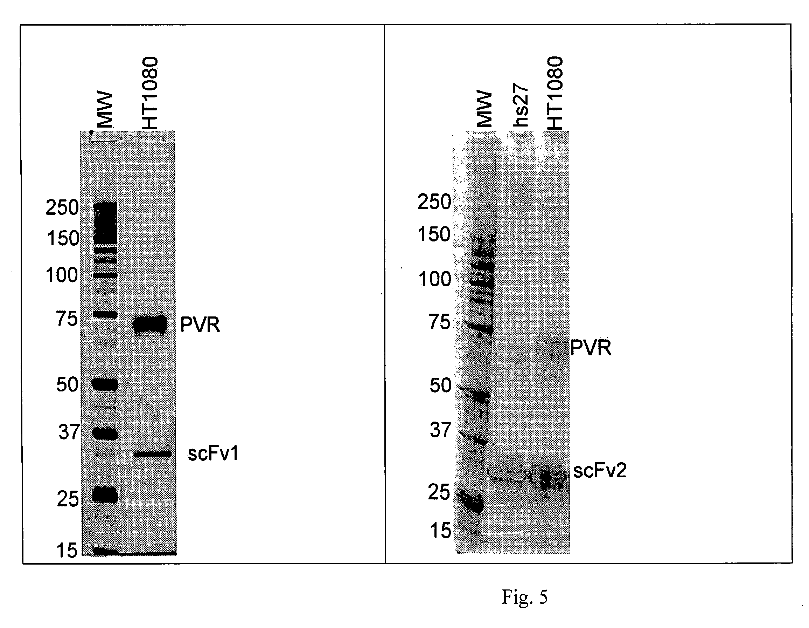

HT1080 and Hs-27 cells (10⁸) were lysed in 3ml 50 mM Tris-HCl, pH 8.0, 150 mM NaCl, 1% Triton X-100 (v/v) containing protease inhibitor cocktail (1 pill in 50ml buffer) (Boehringer Mannheim, Cat.-No. 1697498) and 100 µM Pefablock (Roth, Cat.-No. A154.1). Lysates were pre-incubated for 2h at 4°C with Streptactin-coupled magnetic beads and the supernatants used for the immunoprecipitation reactions. HT1080 specific scFv (50 µg/l mg cell extract) were added to the cleared lysates, samples rotated for 2h at 4°C, placed on a magnet to collect the beads at the tube wall, the beads were washed 4 times with 1ml volume of PBS + 0.1% Tween buffer per wash, before the complexes were isolated by elution from the streptactin magnetic beads with 20 µl 0.1M Citrate pH 3.1. The eluate was immediately neutralized by adding 5 µl 1M Tris-HCl pH 8.0. The immuno-complexes were separated by SDS-PAGE and silver stained for MS analysis.
Both, scFv1 and scFv2, pulled down a protein, detected as a band on SDS-PAGE by silver staining at a molecular weight of about 70 kDa. This band was only detected in the HT1080 cell extract and not in the Hs-27 cells (control cells), see Figure 5.

### Example 14:

### Protein identification via mass spectroscopy

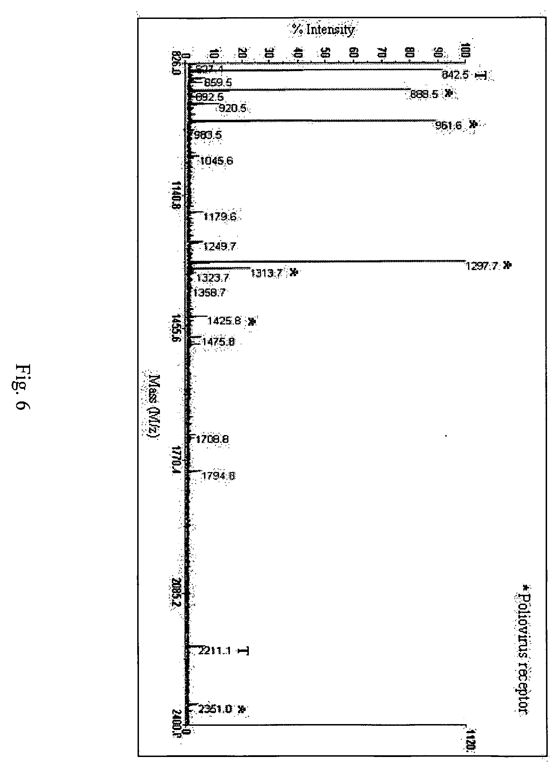

The gel bands obtained from immunoprecipitations followed by SDS PAGE were subjected to a tryptic in-gel digest over night at 37 °C. Peptides were extracted using 5% formic acid and the resulting peptide mixture was desalted using ZipTip µC18 (Millipore) and eluted first with 2 µl of 30% ACN/0.1% TFA, then with 2 µl of 70% ACN/0.1% TFA. The two fractions were pooled and one microliter of the obtained peptide mixtures was mixed in a 1:1 ratio with a solution of α-cyano-4-hydroxycinnamic acid (3 mg/ml), co-crystallized on a Teflon-coated stainless steel target and analyzed on a MALDI-TOF instrument yielding peptide mass fingerprints (PMF) in a mass range of m/z 800-3000. The obtained PMF were used to search all entries for the species *Homo sapiens* in the NCBI and SwissProt databases. In all cases, only peptides matching a given protein with a mass deviation of less than 10 ppm were considered for identification. 4 to 6 peptides were detected, which matched the PVR in the database, with the difference between measured and real mass being always below +/- 10ppm. In some experiments these 4 peptides were observed as 2 doublets, each representing 1 peptide with or without +16 Da, due to methionine oxidation. When a deglycosilation of the peptide mixture was carried out, an additional peptide was observed, which contains Asn120, annotated in the database as being glycosylated. A spectra is shown in Figure 6.

Another sample was also measured by nanoES-MSMS. 4 peptides were detected with this method. Three of them were fragmented by CID (collision induced dissociation) for MSMS analysis. For each fragment a sequence tag was obtained and used for a data base search. Three peptides matched the known poliovirus receptor, one of them with an oxidized Methionine.

### Example 15:

### Methods for epitope mapping

An epitope mapping may be carried out according to one of the following methods:

### Example 15.1: "Classical" epitope mapping

Defined fragments of the cDNA for the antigen of interest are expressed as recombinant fusionproteins or proteins and probed in various assays such as West-emblot or ELISA.

### Example 15.2: Phage display technology

The technique of epitope mapping using random peptide phage display libraries was developed to clone small random fragments of the cDNA for the antigen of interest into the phage protein pIII of filamentous phages and display them on the surface of the phage (Fack et al., (1997) J. Immunol. Methods 7, 43-52). Epitope-displaying phages can be captured with antibodies in a procedure called "biopanning". Sequencing of the inserts of the corresponding phages gives some information on the epitopes. This procedure is used to identify conformational epitopes.

### Example 15.3: Peptide scan technology

It is based on the synthesis of immobilized peptides on activated membranes using the Fmoc chemistry. Amino acid solutions are applied to the activated membrane leading to a peptide bond between the amino-group on the membrane (the membrane is activated with PEG) and the activated carboxy-group of the applied amino acid. After each cycle a specific washing procedure, acetylation, deprotection and monitoring of free amino-groups is performed. In contrast to the in vivo protein-synthesis membrane bound oligo-peptide chains are stepwise synthesized from C- to the N-terminus. Oligo-peptides containing natural as well as modified amino acids can be synthesized up to a length of 20 amino acids. Following synthesis the membranes are equilibrated and unspecific binding sites are blocked. After incubation with the antibody of interest and several washing steps the detection is performed using an HRP-conjugated secondary antibody in combination with the ECL-System. Membranes can be stripped, regenerated, and re-used up to 10 times depending on the antibody. Small overlapping oligo-peptides that ideally cover the complete amino acid sequence of the antigen of interest are synthesized on a solid support. This method is used for the identification of linear epitopes on the amino acid level. It also used for rapid mutational studies.

### Example 16:

### Depletion of cellular PVR protein by RNAi against PVR mRNA

The following ribo-oligonucleotides can be purchased from, e.g. Proligo (Hamburg, Germany, www.proligo.com), Pierce Chemical (part of Perbio Science, Rockford, Illinois, www.perbio.com), or another supplier of RNA oligonucleotides:

1A (5'-CCAGTCACTTGTCTGGAGCTT-3') (SEQ ID No.: 55),

2A (5'-GAGCTTGAAGAAGTGGGTATT-3') (SEQ ID No.: 56),

1B (5'-TGCTGGTGGCATTACTGGTGC-3') (SEQ ID No.: 57),

2B (5'-GCTGTCCTGGCACCCCGTGGAA-3') (SEQ ID No.: 58),

3A (5'-GGCGCGGAGCTGCGGAATGCCT-3') (SEQ ID No.: 59),

4A (5'-CCTCGCTGAGGATGTTCGGGTT-3') (SEQ ID No.: 60)

3B (5'-CCCGTGAACACAGCTGAGGTT-3') (SEQ ID No.: 61),

4B (5'-CAAGGTGGACCCACGAGAGCTTT-3') (SEQ ID No.: 62).

The oligonucleotides 1A and 2A anneal to form siRNA directed against the PVR mRNA, the oligonucleotides 1B and 2B anneal to form a negative control for the pair above, as the nucleotides underlined are mismatches to the PVR mRNA. Also the underlined oligonucleotides 3A and 4A anneal to form siRNA directed against the PVR mRNA, the oligonucleotides 3B and 4B anneal to form a negative control for the 3A/4A-pair, as the nucleotides in bold are mismatches to the PVR mRNA.

The oligonucleotide pairs 1A/2A, 1B/2B, 3A/4A, 3B /4A are annealed using Protocol 2 on page 203 of Elbashir et al., Methods (2002) 26: 199-213. When working with RNA special care is used to avoid RNAse contamination of the oligonucleotides. DEPC-treated water is used for preparing buffers. Gloves are always worn to avoid RNAse-contamination by contact with the skin. For detailed instruction on RNA-related work consult, e.g. Chapter 7.3 and 7.4 of the 2^{nd} edition of Sambrook et al. "Molecular Cloning: A Laboratory Manual" (1989), Cold Spring Harbor Laboratory Press.

HT1080 cells are grown to about 90% confluence in DMEM supplement with Glutamax (Gibco #31966-021) with 10% FCS (Gibco #10270106). 24 h before transfection with the annealed oligonuceotide pairs described above, the 90% confluent cells out of a 175-ml cell culture flask are harvested, e.g. trypsinized with 10ml trypsin-EDTA solution (Life Technologies), gently centrifuged and resuspended in 10ml DMEM. The cell suspension is diluted 1:10 with fresh DMEM medium with 10% FCS without antibiotics and transferred as 500 µl aliquots into each well of a 24-well plate. 24h after seeding the cells a confluency of about 50% is reached.

Separate wells of these cells are then transfected with the annealed oligonucleotide pairs described above according to Protocol 5 on page 207 of Elbashir et al., Methods (2002) 26, 199-213.

After 2 days of incubation according to step 5 of protocol 5 mentioned above, the cells are harvested from the wells and the cells of each well are processed for Western blot, e.g. samples (corresponding to the same number of cells) from a well transfected without oligonucleotides (untreated cells), a well transfected with the oligonucleotide pair 1A/2A, the pair 1B/2B (negative control 1/2), the pair 3A/4A and the pair 3B/4B (negative control 3/4) are loaded on an acrylamide gel, after the run the proteins are blotted onto, e.g. nitrocellulose, e.g. using a semi-dry blotting apparatus, and the nitrocellulose membranes are probed with anti-PVR antibodies.

The amount of PVR protein detected in the samples 1A/2A or 3A/4A is reduced by more than 75% in comparison to the untreated cells. A significant reduction of the level of PVR protein is not detected in the negative controls 1B/2B and 3B/4B in comparison to the untreated cells.

### Example 17:

### Inhibition of adhesion and invasion by RNAi against PVR

HT1080 cells are grown to about 90% confluence in DMEM supplement with Glutamax (Gibco #31966-021) with 10% FCS (Gibco #10270106). 24 h before transfection with the annealed oligonuceotide pairs described above, the 90% confluent cells out of a 175-ml cell culture flask are trypsinized with 10ml trypsin-EDTA solution (Life Technologies). The cell suspension is diluted 1:10 with fresh DMEM medium with 10% FCS without antibiotics and transferred as 500 µl aliquots into each well of a 24-well plate. 24 h after seeding the cells a confluency of about 50% is reached.

Separate wells of these cells are then transfected with the annealed oligonucleotide pairs described above according to protocol 5 on page 207 of Elbashir et al., Methods (2002) 26, 199-213.

After 2 days of incubation according to step 5 of protocol 5 mentioned above, the cells are labeled *in situ* with Bisbenzimide H 33342 (Sigma #B-2261) for 15 min at 37°C, 7,5% CO₂. After washing, the cells are dissociated with 0.5 mM EDTA/PBS (Sigma #E8008), washed and suspended in 0.1% BSA (Sigma #A7030)/DMEM. The cells are then diluted to 6,7 x 10⁵ cells/ml with 0.1% BSA/DMEM, HT1080 cells and added onto the chemotaxis chamber as described in Example 8 or on a 96 well plate as described in Example 11. Invasiveness is then determined as described in Example 8 and adhesiveness is determined as in Example 11.

The adhesiveness or invasiveness measured in at least one of the samples 1A/2A or 3A/4A is reduced by more than 40% in comparison to the untreated cells. A significant reduction of invasiveness is not detected for the negative controls 1B/2B and 3B/4B in comparison to the untreated cells.

## Claims

1. A molecule specifically binding to at least one intra- or extracellular domain of the CD155 (cluster of differentiation 155), the poliovirus receptor (PVR) or. any derivative thereof, wherein the molecule has the ability to detect and/or modulate a receptor mediated adhesion, trafficking and/or invasion behavior of a cell expressing the CD155, the PVR or any derivative thereof.

2. The molecule according to claim 1, wherein the molecule comprises a small chemical compound, oligonucleotide, peptide, oligopeptide, polypeptide, protein, antibody, antibody fragment, anti-idiotypic antibody and/or bioconjugate.

3. The molecule according to claims 1 or 2, wherein the molecule is physically attached to a detectable and/or inducible label.

4. The molecule according to any of the previous claims 1 to 3, wherein the ability to modulate comprises inducing, increasing, stabilizing, strengthening, preventing, inhibiting, decreasing and/or diminishing the adhesion, trafficking and/or invasion behavior of a cell expressing the CD 155, PVR or any derivative thereof.

5. The molecule according to any of the previous claims 1 to 4, wherein the cells expressing the CD 155, the PVR or any derivative thereof are involved in a proliferative disease or disorder, have a metastatic potential or derive from a naturally occurring tumor.

6. The molecule according to any of the previous claims 1 to 5, wherein the molecule, preferably a peptide, polypeptide, antibody, antibody fragment or bioconjugate comprises the amino acid sequence of LWLRRD (SEQ ID No.: 1) and/or WTGDFDY (SEQ ID No.: 2).

7. The molecule according to any of the previous claims 1 to 6, wherein the molecule, preferably a peptide, polypeptide, antibody, antibody fragment or bioconjugate comprises the amino acid sequence of SEQ ID No.: 3, SEQ ID No.: 4, SEQ ID No.: 69 or SEQ ID No.: 70.

8. The molecule according to previous claim 6 or 7, wherein the molecule, preferably a peptide, polypeptide, antibody, antibody fragment or bioconjugate comprises the DNA sequence SEQ ID No.: 5, SEQ ID No.: 6, SEQ ID No.: 71 or SEQ ID No.: 72, or a homologous DNA sequence, or a DNA sequence having a degenerated code but still translating into any of the amino acid sequence according to SEQ ID No.: 3 to SEQ ID No.: 4, SEQ ID No.: 69 or SEQ ID No.: 70.

9. Method of identifying and isolating a molecule according to claim 1 to 8 comprising the steps of:
a) contacting a library of amplifiable ligand-displaying units (ALDUs) with invasive cancer cells;
b) separating said cancer cells and the ALDUs bound thereto from ALDUs not bound to said cells;
c) amplifying the ALDUs bound to said cells;
d) identifying one or more ALDUs and/or ligands derived from an ALDU after step c) which modulates the adhesion, trafficking and/or invasion behavior of the cancer cells;
e) optionally, modifying one or more ALDUs and/or ligands derived from an ALDU after step c) by chemically labeling the ALDUs or ligands and subsequent induction to increase their biological activity as tested in step d);
f) identifying one ore more ALDU and/or ligands derived from an ALDU after step d) or e) specifically binding to CD155, PVR or any derivative thereof;
g) isolating one ore more ALDUs and/or ligands identified in the previous steps; and
h) determining the sequence of isolated ALDUs and/or ligands.

10. Nucleic acid molecule encoding the molecule according to one or several of the previous claims, the ALDU and/or ligand as identified according to the method of claim 9.

11. Vector comprising the nucleic acid molecule according to claim 10.

12. Pharmaceutical composition comprising the molecule according to claims 1 to 8, the molecules or ligands identified according to claim 9, the nucleic acid molecule according to claim 10, the vector according to claim 11 and/or a pharmaceutically acceptable carrier and/or diluent.

13. Use of the molecule according to claims 1 to 8, the molecules or ligands identified according to claim 9, the nucleic acid molecule according to claim 10, the vector according to claim 11 and/or the pharmaceutical composition according to claim 12 as medicament for the prevention and/or treatment of proliferative disorders, cancer or metastasis.

14. Method of modulating *ex vivo* the adhesion behavior of a cell expressing CD155, PVR or any derivative thereof comprising
a) contacting the cell with a molecule according to any of the claims 1 to 8;
b) contacting said cell with a layer of ECM proteins under conditions suitable for the growth of said cells;
c) analyzing the adhesion of said cells to the layer of ECM proteins;
d) optionally, analyzing the adhesion of said cells to the layer of ECM proteins after the chemically modified molecule according to the invention had been induced to increase its biological activity; and
e) determining the percentage of reattachment of cells bound by the unmodified molecule according to the invention and/or cells bound by a modified and induced molecule according to the present invention in comparison with untreated cells.

15. Method of modulating ex vivo the invasion behavior of a cell expressing CD155, PVR or any derivative thereof comprising
a) contacting the cell with a molecule according to any of the claims 1 to 8;
b) contacting said cell with a gel-like matrix under conditions suitable for the growth of said cells;
c) analyzing the migration of said cells through the gel-like matrix;
f) optionally, analyzing the migration of said cells through the gel-like matrix after the chemically modified molecule according to the invention had been induced to increase its biological activity; and
g) determining the percentage of migration of cells bound by the unmodified molecule according to the invention and/or cells bound by a modified and induced molecule according to the present invention in comparison with untreated cells.

16. Method of treating or preventing proliferative disorder or disease, cancer and/or metastasis, said method comprising administering to a patient in need thereof the isolated molecule according to claims 1 to 8, the molecules or ligands identified according to claim 9, the nucleic acid molecule according to claim 10, the vector according to claim 11 and/or the pharmaceutical composition according to claim 12 in an amount effective to modulate the adhesion, trafficking and/or invasion behavior mediated by CD155, PVR or any derivative thereof.

17. A kit comprising the molecule according to claims 1 to 8, the molecules or ligands identified according to claim 9, the nucleic acid molecule according to claim 10, the vector according to claim 11 and suitable testing containers.

18. Method of detecting and/or isolating cells expressing CD155, PVR or any derivative thereof comprising the steps of:
a) contacting the molecule according to claim 6 or 7 with a patient derived cancer cells;
b) separating cells bound to the molecules according to the invention
c) isolating cells bound by the molecules according to the invention; and
d) verifying that the molecules have detected and selected cells expressing PVR.
